# EUROPEAN PATENT APPLICATION

(11) **EP 3 643 779 A1**
(43) Date of publication of application: **29.04.2020**
(21) Application number: 18819665.3
(22) Date of filing: 21.06.2018
(51) Int. Cl.: C12N 15/09, C12N 1/19

(54) **METHOD FOR ASSEMBLING VECTORS WITH HIGH EFFICIENCY IN METHANOL-ASSIMILATING YEAST**

(30) Priority: 23.06.2017 JP 2017122788; 22.12.2017 JP 2017246316
(71) Applicant: Kaneka Corporation, Osaka-shi, Osaka 530-8288 (JP)
(72) Inventor: NISHI Teruyuki, Takasago-shi Hyogo 676-8688 (JP); WATANABE Toru, Takasago-shi Hyogo 676-8688 (JP)
(74) Representative: Hoffmann Eitle
(86) International application number: PCT/JP2018/023679
(87) International publication number: WO 2018/235917

(57) **Abstract**

This invention provides a novel means of inactivating the DNL4 gene associated with non-homologous end joining to completely suppress self-circularization of a vector and assembling vectors efficiently in yeast. By inactivating the DNL4 gene, a methanol-utilizing yeast strain in which non-homologous end joining is suppressed is obtained, and self-circularization of a vector is completely suppressed. Thus, vectors are efficiently assembled in yeast.

## Description

### [Technical Field]

The present invention is related to a method for assembling vectors in a methanol-utilizing yeast strain in which the DNL4 gene associated with non-homologous end joining has been inactivated.

### [Background Art]

In order to artificially engineer microorganisms such as yeast strains for industrial use, in recent years, a various techniques including integration of a gene fragment into the genome, development of an autonomously replicating vector, genome editing, and construction of long-chain artificial chromosomes have been attempted. In genome editing, such as genome integration, however, a target gene may not be integrated into a site of interest, and an unanticipated influence caused by a significantly changed genome structure is an issue of concern. While an autonomously replicating vector that involves a small risk in genome integration has been extensively used in procaryotic/eucaryotic organisms, such as *E*. *coli* or yeast strains, construction of, in particular, long-chain DNA, still remains difficult.

As a means of dissolving the problems described above, a method for assembling vectors in a budding yeast (*Saccharomyces cerevisiae*) using an autonomously replicating vector has been reported (Non-Patent Literature 1). Also, methods for assembling vectors in a type of methanol-utilizing yeast strain that has been used at the industrial level since early times; i.e., *Pichia pastoris,* using an autonomously replicating vector have been reported (Patent Literature 1 and Non-Patent Literatures 2 and 3).

### [Prior Art Literatures]

### [Patent Literatures]

[Patent Literature 1]
WO 2017/055436

### [Non-Patent Literatures]

[Non-Patent Literature 1]
   Nucleic Acids Res. 2009 Nov; 37 (20): 6984-6990
[Non-Patent Literature 2]
   Protein Expr. Purif., 2011 May; 77 (1): 1-8
[Non-Patent Literature 3]
   Microb. Cell Fact., 2016, Aug 11; 15 (1): 139

### [Summary of the Invention]

### [Objects to Be Attained by the Invention]

When assembling vectors in a yeast strain by the methods described in Patent Literature 1 and Non-Patent Literatures 2 and 3, however, other problems and complications, such as self-circularization of a vector caused by non-homologous end joining, low assembly efficiency, selection of pseudopositive strains, and fractional use of selection marker genes for suppressing the background pseudo-positive strains, arise. According to Patent Literature 1, in particular, self-circularization of a vector cannot be suppressed irrespective of the use of a yeast strain in which the KU70 gene associated with non-homologous end joining has been inactivated (Patent Literature 1; Example 9 and Figure 27).

The present invention provides a novel means of completely suppressing self-circularization of a vector to suppress the background pseudo-positive strains at the time of transformation of a methanol-utilizing yeast strain and assembling vectors efficiently in a yeast strain.

### [Means for Attaining the Objects]

The present inventors had conducted extensive analysis on the nucleotide sequence of chromosome DNA of a yeast strain belonging to the genus *Komagataella* to identify the DNL4 protein associated with non-homologous end joining. They also found that self-circularization of a vector could be completely suppressed by inactivating a gene encoding such protein. In addition, they found that vectors could be assembled efficiently in a yeast strain in which the gene was inactivated. This has led to the completion of the present invention.

Specifically, the present invention includes the following.
(1) A method for assembling two or more types of vectors by a transformation method comprising a step of introducing the two or more types of vectors into a methanol-utilizing yeast strain in which the DNL4 gene has been inactivated.
(2) The method according to (1), wherein the DNL4 gene is any of the genes (a) to (d) below:
   (a) a gene comprising the nucleotide sequence as shown in SEQ ID NO: 25;
   (b) a gene comprising a nucleotide sequence of a nucleic acid hybridizing under stringent conditions to a nucleic acid comprising a nucleotide sequence complementary to the nucleotide sequence as shown in SEQ ID NO: 25;
   (c) gene comprising a nucleotide sequence having 85% or higher sequence identity to the nucleotide sequence as shown in SEQ ID NO: 25; and
   (d) a gene encoding an amino acid sequence having 85% or higher sequence identity to the amino acid sequence as shown in SEQ ID NO: 24.
(3) The method according to (1) or (2), wherein the methanol-utilizing yeast strain is a yeast strain belonging to the genus *Komagataella* or a yeast strain belonging to the genus *Ogataea.*
(4) The method according to any of (1) to (3), wherein the two or more types of vectors each comprise a nucleotide sequence having 85% or higher sequence identity to one another.
(5) The method according to any of (1) to (4), wherein at least one of the two or more types of vectors comprises an autonomously replicating sequence (ARS).
(6) The method according to any of (1) to (5), wherein at least one of the two or more types of vectors is an autonomously replicating vector.
(7) The method according to (6), wherein the autonomously replicating vector further comprises an autonomously replicating sequence (ARS) and/or a centromeric DNA sequence derived from a yeast strain belonging to the genus *Komagataella* or a yeast strain belonging to the genus *Ogataea.*
(8) A method for producing assembled vectors comprising the method according to any of (1) to (7).
(9) Assembled vectors obtained by the method according to any of (1) to (8).
(10) A method for producing a transformant comprising a step of transformation with the assembled vectors obtained by the method according to any of (1) to (8).
(11) A transformant transformed with the assembled vectors according to (9).

The present specification encompasses the content disclosed in JP Patent Application Nos. 2017-122788 and 2017-246316 to which present application claims priority.

### [Advantageous Effects of Invention]

The present invention can suppress development of background pseudo-positive strains in transformation of a methanol-utilizing yeast strain by completely suppressing self-circularization of a vector. In addition, the present invention provides a novel means for assembling vectors efficiently in a yeast strain.

### [Brief Description of the Drawings]

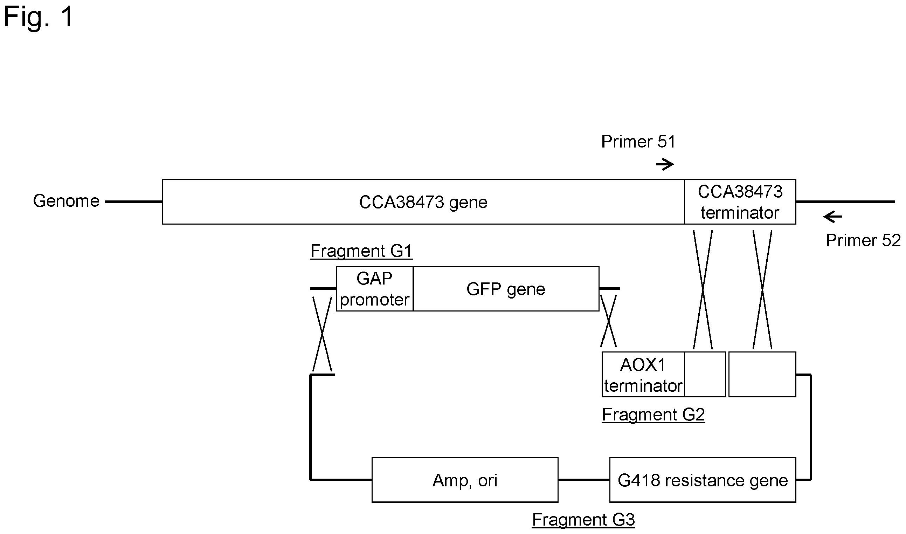

Fig. 1 schematically shows the constitutions of vector fragments used for genome integration used in Production Example 7, Comparative Example 4, and Example 5.

### [Description of Embodiment]

Hereinafter, the present invention is described in detail with reference to preferable embodiments.

The term "non-homologous end joining" used herein refers to a mechanism in which ends of the cleaved DNA double strands are bound to each other. For example, KU70 and KU80 dimers recognize the ends and a DNL4-LIF1 complex as a ligase binds the ends to each other.

A gene associated with non-homologous end joining is a gene of a protein associated with the mechanism described above, and examples thereof include KU70, KU80, DNL4, and LIF1 genes. In the case of the *Komagataella pastoris* strain ATCC76273, for example, the KU70 gene is represented by a nucleotide sequence encoding a polypeptide as shown in Accession No. CCA39840 (KU70), the KU80 gene is represented by a nucleotide sequence encoding a polypeptide as shown in Accession No. CCA40385 (KU80), and the DNL4 gene is represented by a nucleotide sequence encoding a polypeptide as shown in Accession No. CCA39424 (DNL4).

According to an embodiment of the present invention, the DNL4 gene is any of the genes (a) to (d) below:
(a) a gene comprising the nucleotide sequence as shown in SEQ ID NO: 25;
(b) a gene comprising a nucleotide sequence of a nucleic acid hybridizing under stringent conditions to a nucleic acid comprising a nucleotide sequence complementary to the nucleotide sequence as shown in SEQ ID NO: 25;
(c) a gene comprising a nucleotide sequence having 85% or higher sequence identity to the nucleotide sequence as shown in SEQ ID NO: 25; and
(d) a gene encoding an amino acid sequence having 85% or higher sequence identity to the amino acid sequence as shown in SEQ ID NO: 24.

When two nucleic acids hybridize to each other under stringent conditions in the present invention, for example, nucleic acid Y is considered as "the nucleic acid that hybridizes under stringent conditions to the nucleic acid X" when the nucleic acid Y can be obtained as the nucleic acid bound on a filter by using the filter with an immobilized nucleic acid X, hybridizing it to a nucleic acid Y exhibiting sequence identity of 85% or higher at 65°C in the presence of 0.7 to 1.0 M NaCl, and then washing the filter under the condition of 65°C using a 2-fold SSC solution (the composition of 1-fold SSC solution consists of 150 mM sodium chloride and 15 mM sodium citrate). Alternatively, the nucleic acid X and the nucleic acid Y can be said to "hybridize to each other under stringent conditions." As the concentration of the SSC solution is lowered, hybridization of nucleic acids with higher sequence identity can be expected. Accordingly, the nucleic acid Y can be obtained in the form of the nucleic acid bound on a filter when the filter is washed preferably at 65°C using a 1-fold SSC solution, more preferably at 65°C using a 0.5-fold SSC solution, still more preferably at 65°C using a 0.2-fold SSC solution, and further preferably at 65°C using a 0.1-fold SSC solution. In addition, hybridization of nucleic acids with higher sequence identity can be expected as temperature increases. Accordingly, the nucleic acid Y can be obtained in the form of the nucleic acid bound on a filter when the filter is washed preferably at 70°C using a 2-fold SSC solution, more preferably at 75°C using a 2-fold SSC solution, still more preferably at 80°C using a 2-fold SSC solution, and further preferably at 85°C using a 2-fold SSC solution. The standard nucleic acid X may be a colony- or plaque-derived nucleic acid X.

The sequence identity of a nucleotide sequence and an amino acid sequence in the present invention can be determined by a method or with the use of sequence analysis software known to a person skilled in the art. Examples include the Blastn program and Blastp program of BLAST algorithm, and Fasta program of FASTA algorithm. In the present invention, the "sequence identity" of a certain nucleotide sequence to be evaluated to a nucleotide sequence X is a value shown in % of the frequency with which the same nucleotides appear in the same sites of the nucleotide sequences including the gap portions, when the nucleotide sequence X and the nucleotide sequence to be evaluated are aligned and gaps are introduced as needed to achieve the highest nucleotide alignment between both sequences. In the present invention, the "sequence identity" of a certain amino acid sequence to be evaluated to an amino acid sequence X is a value shown in % of the frequency with which the same amino acid appears in the same site of the amino acid sequences including the gap portions, when the amino acid sequence X and the amino acid sequence to be evaluated are aligned and gaps are introduced as needed to achieve the highest amino acid alignment between both sequences.

The sequence identity between the gene indicated above and the nucleotide sequence as shown in SEQ ID NO: 25 is 85% or higher, preferably 90% or higher, more preferably 95% or higher, further preferably 96% or higher, particularly preferably 97% or higher, 98% or higher, and most preferably 99% or higher.

According to the present invention, a "nucleic acid" may also be referred to as a "polynucleotide," it refers to DNA or RNA, and it typically refers to DNA. Double-stranded or single-stranded DNA may be used, DNA comprising a given nucleotide sequence may be double-stranded DNA comprising the given nucleotide sequence in one strand, single-stranded DNA comprising the given nucleotide sequence (a sense strand), or single-stranded DNA comprising a sequence complementary to the given nucleotide sequence (an anti-sense strand).

The term "a nucleotide sequence encoding an amino acid sequence" used herein refers to a nucleotide sequence designed based on a codon table to a polypeptide consisting of an amino acid sequence, and such nucleotide sequence produces a polypeptide by transcription and translation.

In the present invention, the "polypeptide" comprises 2 or more amino acids peptide-bonded to one another, and it encompasses a short-chain substance referred to as a peptide or oligopeptide in addition to a protein.

In the present invention, the "gene" encompasses DNA and its transcript, mRNA, unless otherwise specified. It is typically DNA, and it is particularly preferably DNA included in a host chromosome. In an embodiment in which a gene is mRNA, T (thymine) in a given nucleotide sequence (e.g., SEQ ID NO: 25) may be read as U (uracil). The term "gene" used herein refers to any of a regulatory region, a coding region, an exon, and an intron, unless otherwise specified.

When a gene is "inactivated" in the present invention, gene functions are lost or attenuated. When a gene is "inactivated," the expression level of a transcript of such gene, mRNA, or a translation product, polypeptide, is low, or mRNA or a protein does not normally function. The mRNA expression level can be quantified by, for example, real-time PCR, RNA-Seq, Northern hybridization, or DNA array-based hybridization. The polypeptide expression level can be quantified with the use of, for example, an antibody that recognizes a polypeptide or a dye compound that can bind to a polypeptide. In addition to the quantification methods mentioned above, a conventional technique employed by a person skilled in the art may be employed herein.

A gene can be inactivated by, for example, DNA mutation using an agent or ultraviolet rays, site-directed mutagenesis using PCR, RNAi, a protease, or homologous recombination. When a gene is to be inactivated, a nucleotide sequence in an ORF of the target gene may be modified (via deletion, substitution, addition, or insertion) and/or a nucleotide sequence in a region that regulates initiation or termination of transcription, such as a promoter region, an enhancer region, or a terminator region, may be modified (via deletion, substitution, addition, or insertion). A site to be subjected to deletion, substitution, addition, or insertion or a nucleotide sequence to be subjected to deletion, substitution, addition, or insertion is not particularly limited, provided that normal functions of the target gene can be deleted. A gene to be inactivated may be located inside or outside the chromosome of a methanol-utilizing yeast strain. The gene located inside the chromosome is preferably inactivated. Typically, the DNL4 gene can be inactivated by deleting at least a part of the gene coding region in the host chromosome (e.g., a nucleotide sequence region comprising the number of nucleotides that is 50% or more, preferably 60% or more, more preferably 70% or more, still more preferably 80% or more, further preferably 90% or more, and most preferably 100% relative to the number of nucleotides constituting the nucleotide sequence of the coding region).

In the present invention, "modification of a nucleotide sequence" can be carried out by a technique, such as gene insertion via homologous recombination or site-directed mutagenesis. For example, an upstream promoter may be substituted with a promoter with lower activity, a codon may be modified into another codon that is not suitable for a methanol-utilizing yeast strain, or a vector comprising an upstream sequence of the gene to be deleted, a selection marker gene sequence, and a downstream sequence of the gene to be deleted ligated thereto may be introduced.

In the present invention, an extent of lowering in the expression level is not particularly limited, and an extent of lowering is preferably 5% or more, 10% or more, 20% or more, 30% or more, 40% or more, 50% or more, 60% or more, 70% or more, 80% or more, 90% or more, or 95% or more. It is well-known to a person skilled in the art that the gene can be inactivated even if the expression level is not lowered, provided that normal functions of a gene product can be deleted as described above. Thus, the extent of lowering in the expression level is merely a criterion for inactivation.

The number of inactivated DNL4 genes is not particularly limited. In the methanol-utilizing yeast strain of the present invention, other genes associated with non-homologous end joining may further be inactivated. For example, dual inactivation of the KU70 gene and DNL4 gene, KU80 gene and DNL4 gene, or DNL4 gene and LIF1 gene, triple inactivation of KU70 gene, KU80 gene, and DNL4 gene, KU70 gene, DNL4 gene, and LIF1 gene, or KU80 gene, DNL4 gene, and LIF1 gene, or quadruple inactivation of KU70 gene, KU80 gene, DNL4 gene, and LIF1 gene may be employed.

The "gene comprising the nucleotide sequence as shown in SEQ ID NO: 25" was found as a result of extensive analysis of the nucleotide sequences of 4 chromosome DNAs of *Komagataella pastoris* (the strain ATCC76273: Accession Nos. FR839628 to FR839631 (J. Biotechnol., 154 (4), 312-320, 2011); and the strain GS115: Accession Nos. FN392319 to FN392322 (Nat. Biotechnol., 27 (6), 561-566, 2009)). Specifically, the present inventors searched for a polypeptide capable of suppressing self-circularization of a vector via inactivation and a polynucleotide comprising a nucleotide sequence encoding the amino acid sequence of the polypeptide. As a result, they found a polypeptide comprising an amino acid sequence (Accession No. CCA39424) as shown in SEQ ID NO: 24 (DNL4) and a polynucleotide comprising a nucleotide sequence as shown in SEQ ID NO: 25 encoding such polypeptide in the strain ATCC76273. In the examples described below, the present inventors completed a method that enables efficient vector assembly in yeast with the use of, as a host cell, a methanol-utilizing yeast strain in which a gene comprising the nucleotide sequence has been inactivated.

In a method for inactivating the gene comprising the nucleotide sequence as shown in SEQ ID NO: 25 (i.e., the DNL4 gene), for example, a nucleotide sequence exhibiting 100% sequence identity to a 1,000-bp promoter located upstream of the gene comprising the nucleotide sequence as shown in SEQ ID NO: 25 (e.g., the nucleotide sequence as shown in SEQ ID NO: 1) and a nucleotide sequence exhibiting 100% sequence identity to a 1,007-bp terminator located downstream of the gene comprising the nucleotide sequence as shown in SEQ ID NO: 25 (e.g., the nucleotide sequence as shown in SEQ ID NO: 2) are prepared as PCR products with the use of primers 1 to 4, as described in the examples below. A vector comprising the PCR products is transformed into a yeast strain belonging to the genus *Komagataella,* and homologous recombination may be carried out with the use of the selected strain. Thus, the gene of interest can be deleted.

The "gene encoding an amino acid sequence having 85% or higher sequence identity to the amino acid sequence as shown in SEQ ID NO: 24" comprises a nucleotide sequence designed with reference to a codon table based on a polypeptide comprising an amino acid sequence having 85% or higher sequence identity to the amino acid sequence as shown in SEQ ID NO: 24. An example of such gene is the gene as shown in SEQ ID NO: 25. By inactivating such gene, self-circularization of a vector can be suppressed, and vectors can be assembled efficiently in a yeast strain. The sequence identity is 85% or higher, preferably 90% or higher, more preferably 95% or higher, further preferably 96% or higher, particularly preferably 97% or higher, 98% or higher, and most preferably 99% or higher.

A methanol-utilizing yeast strain of the present invention in which the DNL4 gene has been inactivated is preferably obtained by transforming a methanol-utilizing yeast strain with a vector comprising at least one of a nucleotide sequence having a homologous region to that of the DNL4 gene, a nucleotide sequence comprising a homologous region to that of the DNL4 gene promoter, and a nucleotide sequence comprising a homologous region to a DNL4 gene terminator.

The methanol-utilizing yeast strain of the present invention in which the DNL4 gene has been inactivated is preferably obtained by transforming a methanol-utilizing yeast strain with a vector comprising at least one of, for example, a nucleotide sequence having a homologous region to that of any of the genes (a) to (d) above, a nucleotide sequence comprising a homologous region to that of the promoter of any of the genes (a) to (d) above, and a nucleotide sequence comprising a homologous region to that of the terminator of any of the genes (a) to (d) above.

In the present invention, the DNL4 gene in the chromosome of the methanol-utilizing yeast strain, such as any of the genes (a) to (d) above, can be inactivated via transformation.

In a particularly preferable embodiment in which the DNL4 gene, which is any of the genes (a) to (d) above, is inactivated, a DNA fragment 1 comprising a nucleotide sequence having 85% or higher sequence identity to a partial nucleotide sequence of the nucleotide sequence of the promoter located upstream of the gene in the host chromosome (i.e., a partial nucleotide sequence 1) and a DNA fragment 2 comprising a nucleotide sequence having 85% or higher sequence identity to a partial nucleotide sequence of the nucleotide sequence of the terminator located downstream of the gene (i.e., a partial nucleotide sequence 2) are ligated to each other, so that the DNA fragment 1 is located upstream and the DNA fragment 2 is located downstream, so as to construct a vector. The resulting vector is transformed into a host, and the gene of interest is deleted via homologous recombination. The vector is preferably a linear vector prepared by cleaving a cyclic vector comprising the DNA fragment 1 and the DNA fragment 2 at the restriction enzyme recognition site inside the DNA fragment 1 or the DNA fragment 2 and linearizing the resultant. An example of the nucleotide sequence of the promoter is the nucleotide sequence as shown in SEQ ID NO: 1. An example of the nucleotide sequence of the terminator is the nucleotide sequence as shown in SEQ ID NO: 2. The length of the partial nucleotide sequence 1 and that of the partial nucleotide sequence 2 are not particularly limited, as long as homologous recombination can be implemented. The length is preferably 100 bp or longer, 200 bp or longer, 300 bp or longer, 400 bp or longer, 500 bp or longer, 600 bp or longer, 700 bp or longer, 800 bp or longer, 900 bp or longer, or 1,000 bp or longer. The sequence identity of 85% or higher mentioned above is preferably 90% or higher, more preferably 95% or higher, more preferably 96% or higher, more preferably 97% or higher, more preferably 98% or higher, and more preferably 99% or higher.

In the present invention, the methanol-utilizing yeast is defined as a yeast which can be cultured utilizing methanol as an only carbon source, but yeast which was originally a methanol-utilizing yeast but lost the methanol-utilizing ability due to an artificial modification or mutation is also within the scope of the methanol-utilizing yeast of the present invention.

Examples of the methanol-utilizing yeast strains include yeast strains belonging to the genus *Pichia,* the genus *Ogataea,* the genus *Candida,* the genus *Torulopsis,* and the genus *Komagataella.* Preferable examples include *Pichia methanolica* in the genus *Pichia, Ogataea angusta, Ogataea polymorpha, Ogataea parapolymorpha,* and *Ogataea minuta* in the genus *Ogataea, Candida boidinii* in the genus *Candida,* and *Komagataella pastoris* and *Komagataella phaffii* in the genus *Komagataella.*

Among the methanol-utilizing yeast strains described above, yeast strains belonging to the genus *Komagataella* or yeast strains belonging to the genus *Ogataea* are particularly preferable.

As yeast strains belonging to the genus *Komagataella, Komagataella pastoris* and *Komagataella phaffii* are preferable. Both *Komagataella pastoris* and *Komagataella phaffii* are also referred to as *"Pichia pastoris."*

Specific examples of strains that can be used as hosts include *Komagataella pastoris* ATCC76273 (Y-11430, CBS7435) and *Komagataella pastoris* X-33. These strains are available from American Type Culture Collection or Thermo Fisher Scientific, Inc.

As yeast strains belonging to the genus *Ogataea, Ogataea angusta, Ogataea polymorpha,* and *Ogataea parapolymorpha* are preferable. These 3 strains are closely related to each other and are also referred to as *"Hansenula polymorpha"* or *"Pichia angusta."*

Specific examples of strains that can be used include *Ogataea angusta* NCYC495 (ATCC14754), *Ogataea polymorpha* 8V (ATCC34438), and *Ogataea parapolymorpha* DL-1 (ATCC26012). These strains are available from, for example, American Type Culture Collection.

In the present invention, in addition, derivative strains from the yeast strains belonging to the genus *Komagataella* or the genus *Ogataea* can also be used, an example of a histidine-auxotrophic yeast strain is the *Komagataella pastoris* GS115 strain (available from Thermo Fisher Scientific, Inc.), and examples of a leucine-auxotrophic yeast strains include NCYC495-derived BY4329, 8V-derived BY5242, and DL-1-derived BY5243 (these can be distributed from National BioResource Project). In the present invention, derivative strains from these strains can also be used.

The vector of the present invention (at least one of the two or more types of vectors used in the present invention and/or a vector formed of two or more types of vectors assembled to each other) can be a cyclic vector, a linear vector, a plasmid vector, or an artificial chromosome vector.

The "vector" in the present invention is a nucleic acid molecule that is artificially constructed. Nucleic acid molecules constituting the vector of the present invention (the two or more types of vectors used in the present invention and/or a vector formed of two or more types of vectors assembled to each other) is generally DNA, and it is preferably double-stranded DNA. A cyclic or linear DNA may be used. At least one of the two or more types of vectors and a vector formed of two or more types of vectors assembled to each other used in the present invention can generally comprise a cloning site including 1 or more restriction enzyme recognition sites, an overlapping region for utilizing an In-Fusion cloning system of Clontech Laboratories, Inc. or a Gibson Assembly system of New England Biolabs, a nucleotide sequence of an endogenous gene, a nucleotide sequence encoding a polypeptide comprising the amino acid sequence of the target protein, and a nucleotide sequence of a selectable marker gene (e.g., an auxotrophic complementary gene and a drug resistance gene). Examples of linear vectors include auxotrophic complementary genes, such as URA3 gene, LEU2 gene, ADE1 gene, HIS4 gene, and ARG4 gene, PCR products comprising nucleotide sequences of drug resistant genes, such as G418 resistance gene, Zeocin (tradename) resistance gene, hygromycin resistance gene, Clone NAT resistance gene, and blasticidin S resistance gene, and linear vectors prepared by cleaving a cyclic or plasmid vector with an adequate restriction enzyme. Examples of plasmid vectors that can be used include YEp vector, YRp vector, YCp vector, pPICHOLI (http: //www.mobitec.com/cms/products/bio/04_vector_sys/p_picholi_shuttle_vector.html), pHIP (Journal of General Microbiology, 1992, 138, 2405-2416, Chromosomal targeting of replicating plasmids in the yeast *Hansenula polymorpha*), pHRP (see the documents referred concerning pHIP above), pHARS (Molecular and General Genetics MGG February 1986, Volume 202, Issue 2, pp. 302-308, Transformation of the methylotrophic yeast *Hansenula polymorpha* by autonomous replication and integration vectors), *E*. *coli*-derived plasmid vectors (pUC18, pUC19, pBR322, pBluescript, and pQE), and *Bacillus subtilis*-derived plasmid vectors (pHY300PLK and pMTLBS72). In general, examples of artificial chromosome vectors include artificial chromosome vectors comprising centromeric DNA sequence, telomeric DNA sequence, and autonomously replicating sequence (ARS). In the case of yeast, an example is an yeast artificial chromosome (YAC) vector. Such vectors have been developed in, for example, *Saccharomyces cerevisiae* or *Schizosaccharomyces pombe.* In the case of *Komagataella pastoris,* an artificial chromosome vector can be constructed with the use of the centromeric DNA sequence described in WO 2016/088824.

In the present invention, the term "transformation" refers to introduction of the vector into the methanol-utilizing yeast strain. Introduction of a vector into a methanol-utilizing yeast strain; i.e., transformation, can be adequately carried out via a known technique, and examples thereof include, but are not particularly limited to, the electroporation method, the lithium acetate method, and the spheroplast method. For example, the electroporation method described in High efficiency transformation by electroporation of *Pichia pastoris* pretreated with lithium acetate and dithiothreitol (Biotechniques, Jan. 2004; 36 (1): 152-4) is a common transformation method for *Komagataella pastoris.*

When a methanol-utilizing yeast strain is transformed with a vector in the present invention, a selectable marker gene such as an auxotrophic complementary gene or a drug resistance gene is preferably used. A selectable marker is not particularly limited. In the case of a methanol-utilizing yeast strain, an auxotrophic complementary gene such as URA3 gene, LEU2 gene, ADE1 gene, HIS4 gene, or ARG4 gene enables selection of a transformant by the recovery of a prototrophic phenotype in auxotrophic strains of uracil, leucine, adenine, histidine, and arginine, respectively. When a vector comprising a drug resistance gene such as G418 resistance gene, Zeocin (tradename) resistance gene, hyglomycin resistance gene, Clone NAT resistance gene, or blasticidin S resistance gene is used, alternatively, the transformant can be selected based on the drug resistance on the relevant medium containing G418, Zeocin (tradename), and hyglomycin, Clone NAT, or blasticidin S, respectively. It is not possible to use an auxotrophic selectable marker used for preparing a transformant when such selectable marker is not disrupted in the host. In such a case, the selectable marker may be disrupted in the host, and a method known to a person skilled in the art can be employed.

According to the method of transformation of the present invention, vectors can be efficiently assembled in yeast. Thus, the method comprises a step of introducing two or more types of vectors. Preferably, two or more types of vectors are introduced successively or simultaneously. For example, competent cells are mixed with two or more types of vectors before the process of transformation as performed in the present examples.

When transformation is carried out with the use of two or more types of vectors in the present invention, it is preferable that vectors be constructed to comprise nucleotide sequences exhibiting sequence identity to each other as described in the examples below. It is more preferable that each of two or more types of vectors comprise, as a partial nucleotide sequence, a nucleotide sequence exhibiting sequence identity to that of at least an other vector. In the nucleotide sequences, the number of sites exhibiting sequence identity may be 1, or it may be 2 or more.

In the "nucleotide sequences exhibiting sequence identity to each other" according to the present invention, at least a part of one nucleotide sequence is identical to that of the other nucleotide sequence. Such nucleotide sequences exhibit sequence identity of preferably 85% or higher, more preferably 90% or higher, further preferably 95% or higher, and most preferably 100%. The length of such nucleotide sequences exhibiting sequence identity is not particularly limited, provided that it is 20 bp or longer. Thus, two vectors comprising such nucleotide sequences can be assembled. Specifically, the length of nucleotide sequences is preferably 20 bp or longer, 30 bp or longer, 40 bp or longer, 50 bp or longer, 60 bp or longer, 70 bp or longer, 80 bp or longer, 90 bp or longer, 100 bp or longer, 200 bp or longer, 300 bp or longer, 400 bp or longer, 500 bp or longer, or 1,000 bp or longer.

When transformation is carried out with the use of two or more types of vectors in the present invention, the molar ratio thereof is not particularly limited. When two types of vectors are used, for example, the molar ratio is preferably 1:1, 1:1 or more, 1:2 or more, 1:3 or more, 1:4 or more, 1:5 or more, 1:10 or more, 1:20 or more, 1:30 or more, 1:40 or more, or 1:50 or more.

The term "assembly" used in the present invention refers to a mechanism in which two or more types of vectors introduced into a yeast strain are ligated to each other with the aid of nucleotide sequences exhibiting sequence identity to each other.

According to a preferable embodiment, each of two or more types of vectors comprises, as a partial nucleotide sequence, a nucleotide sequence exhibiting sequence identity to that of at least an other vector, the two or more types of vectors are assembled to each other via homologous recombination between such partial nucleotide sequences, and one linear or cyclic vector is constructed as a consequence. The one linear vector may be integrated into the host genome DNA. In such a case, one of the two or more types of vectors further comprises, as a partial nucleotide sequence, a nucleotide sequence exhibiting sequence identity (preferably 85% or higher, more preferably 90% or higher, further preferably 95% or higher, and most preferably 100%) to the nucleotide sequence located upstream of the site where the vector has been inserted into the host genome DNA. The other of the two or more types of vectors further comprises, as a partial nucleotide sequence, a nucleotide sequence exhibiting sequence identity (preferably 85% or higher, more preferably 90% or higher, further preferably 95% or higher, and most preferably 100%) to the nucleotide sequence located downstream of the site where the vector has been inserted into the host genome DNA.

The "host cell" in the present invention refers to a cell to be transformed by introducing a vector thereinto. A host cell after transformation is occasionally referred to as the "transformant" herein. The cell used as the host is not particularly limited, as long as it is a methanol-utilizing yeast cell into which a vector can be introduced.

The vector of the present invention (at least one of the two or more types of vectors used in the present invention and/or a vector formed of two or more types of vectors assembled to each other) can comprise an autonomously replicating sequence (ARS). In the present invention, ARS is a replication origin in methanol-utilizing yeast strains, such as prokaryotes (e.g., *E. coli,* bacteria, actinomycete, Eubacteria, archaebacteria, or blue-green algae), viruses (e.g., DNA viruses or RNA viruses), and eukaryotes (e.g., fungi, algae, protozoan, yeast, plants, animals, birds, fowls, mammals, humans, or mice), preferably in eukaryotes, and more preferably in yeast, such as *Komagataella pastoris.* It is a region in a nucleotide sequence where replication is initiated. The vector of the present invention may comprise two or more ARSs of, for example, different yeast species. An example of ARS that can be within the scope of the vector of the present invention is a centromeric DNA sequence comprising ARS in *Komagataella pastoris* comprising the nucleotide sequence as shown in SEQ ID NO: 6 described in the examples below. This sequence is a centromeric DNA sequence of chromosome DNA 2 of *Komagataella pastoris* (Accession No. FR839629).

Another example of ARS that can be within the scope of the vector of the present invention (at least one of the two or more types of vectors used in the present invention and/or a vector formed of two or more types of vectors assembled to each other) is PARS1, which is ARS in *Komagataella pastoris* comprising the nucleotide sequence as shown in SEQ ID NO: 37, as described in the examples below. This sequence is a 164-bp ARS located in a region between position 1980709 and position 1980872 of chromosome DNA 2 of *Komagataella pastoris* (Accession No. FR839629).

The vector of the present invention (at least one of the two or more types of vectors used in the present invention and/or a vector formed of two or more types of vectors assembled to each other) may not comprise an autonomously replicating sequence (ARS). For example, vectors assembled in yeast may be integrated into the host chromosome. According to a preferable embodiment, two or more types of vectors used in the present invention are each a nucleic acid fragment incapable of autonomous replication, and a vector formed of the two or more types of vectors assembled to each other is integrated into the host genome DNA. According to a more preferable embodiment, two or more types of vectors used in the present invention are each a nucleic acid fragment incapable of autonomous replication, and the two or more types of vectors are integrated into the host genome DNA via homologous recombination in the host genome DNA region exhibiting sequence identity to the upper terminal and/or lower terminal sequence(s) of the vector formed of the two or more types of vectors assembled to each other.

The vector of the present invention (at least one of the two or more types of vectors used in the present invention and/or a vector formed of two or more types of vectors assembled to each other) is preferably an autonomously replicating vector. When the vector of the present invention is an autonomously replicating vector, the host cell can be modified without changing the genome sequence or genome structure of the host cell. According to a particularly preferable embodiment, one of the two or more types of vectors used in the present invention is an autonomously replicating vector, the other of the two or more types of vectors is a nucleic acid fragment incapable of autonomous replication by itself, and a vector formed of the two or more types of vectors assembled to each other is an autonomously replicating vector.

In the present invention, an autonomously replicating vector is replicated independently of a host chromosome, and such vector is replicated in the host cell without being integrated into the host chromosome.

The vector according to this embodiment of the present invention can be used in the form of an autonomously replicating vector in a methanol-utilizing yeast strain, such as a yeast strain belonging to the genus *Komagataella* or a yeast strain belonging to the genus *Ogataea,* and preferably in *Komagataella pastoris.* Specifically, the autonomously replicating vector preferably comprises ARS and/or a centromeric DNA sequence derived from a methanol-utilizing yeast strain, and it more preferably comprises ARS and/or a centromeric DNA sequence derived from a yeast strain belonging to the genus *Komagataella* or a yeast strain belonging to the genus *Ogataea.*

The vector of the present invention (at least one of the two or more types of vectors used in the present invention and/or a vector formed of two or more types of vectors assembled to each other) is preferably an autonomously replicating vector that is capable of autonomous replication in host cells of a plurality of organism species. An example of such autonomously replicating vector is a vector comprising ARS and/or a centromeric DNA sequence derived from the yeast strain belonging to the genus *Komagataella* or the yeast strain belonging to the genus *Ogataea* and ARS and/or a centromeric DNA sequence derived from an organism species other than the organism species from which the aforementioned ARS and/or centromeric DNA sequence are/is derived. Specific examples of means that can be adopted to make the vector of the present invention to autonomously replicate in hosts of other species or genera, as well as in *Komagataella pastoris* include: a means of cloning a centromeric DNA sequence of the human chromosome into a vector comprising a centromeric DNA sequence including ARS in *Komagataella pastoris* comprising the nucleotide sequence as shown in SEQ ID NO: 6 to prepare a human artificial chromosome; a means of cloning ARS or a centromeric DNA sequence of a yeast strain belonging to the genus *Ogataea* into a vector comprising the nucleotide sequence as shown in SEQ ID NO: 6 to prepare an autonomously replicating vector that can be used in the both genera; a means of cloning ARS or a centromeric DNA sequence of a budding yeast (*Saccharomyces cerevisiae*) or a fission yeast (*Schizosaccharomyces pombe*) into the vector comprising the nucleotide sequence as shown in SEQ ID NO: 6 to prepare an autonomously replicating vector that can be used in both the genera; and a means of cloning genes encoding proteins constituting the centromere of *Komagataella pastoris* into a vector comprising the nucleotide sequence as shown in SEQ ID NO: 6 to prepare an autonomously replicating vector in other genera. As described in the examples, the vector can be used in combination with an autonomously replicating vector in *E*. *coli.*

In the present invention, a centromeric DNA sequence is a nucleotide sequence that forms a structure referred to as a "kinetochore" to which a spindle binds. In humans, for example, it is a region where a long arm of the chromosome intersects with a short arm thereof, and it is located substantially in the middle of the chromosome. This region is accordingly referred to as a centromeric region.

Hereafter, the present invention is described in detail with reference to Production Examples, Comparative Examples, and Examples, although the present invention is not limited to these examples.

### < Production Example 1: Preparation of various genes used for preparing vectors>

A detailed engineering method and the like related to recombinant DNA technology used in the Examples below are described in the following books: Molecular Cloning 2nd Edition (Cold Spring Harbor Laboratory Press, 1989) and Current Protocols in Molecular Biology (Greene Publishing Associates and Wiley-Interscience).

In the Examples below, a plasmid used for transforming a yeast was prepared by introducing a constructed vector into an *Escherichia coli* (*E. coli*) HST08 competent cells (Takara Bio Inc.), culturing the obtained transformants to amplify the plasmid. Preparation of the plasmid from the strain carrying the plasmid was performed by using FastGene Plasmid Mini Kit (NIPPON Genetics Co., Ltd.).

A DNL4 promoter (SEQ ID NO: 1), a DNL4 terminator (SEQ ID NO: 2), a promoter-regulated ADE1 gene sequence (SEQ ID NO: 3), a GAP promoter (SEQ ID NO: 4), a GAP1 terminator (SEQ ID NO: 5), a centromeric DNA sequence (SEQ ID NO: 6), and PARS1 (SEQ ID NO: 37) were used for vector construction. These sequences were prepared by PCR using, as a template, a mixture of chromosome DNA from the *Komagataella pastoris* strain ATCC76273 (the nucleotide sequences thereof are described in EMBL (The European Molecular Biology Laboratory) Accession Nos. FR839628 to FR839631). The DNL4 promoter was prepared by PCR using primer 1 (SEQ ID NO: 7) and primer 2 (SEQ ID NO: 8). The DNL4 terminator was prepared by PCR using primer 3 (SEQ ID NO: 9) and primer 4 (SEQ ID NO: 10). The promoter-regulated ADE1 gene sequence was prepared by PCR using primer 5 (SEQ ID NO: 11) and primer 6 (SEQ ID NO: 12). The GAP promoter was prepared by PCR using primer 7 (SEQ ID NO: 13) and primer 8 (SEQ ID NO: 14). The GAP1 terminator was prepared by PCR using primer 9 (SEQ ID NO: 15) and primer 10 (SEQ ID NO: 16). The centromeric DNA sequence was prepared by PCR using primer 11 (SEQ ID NO: 17), primer 12 (SEQ ID NO: 18), primer 13 (SEQ ID NO: 19), and primer 14 (SEQ ID NO: 20). PARS1 was prepared by PCR using primer 26 (SEQ ID NO: 38) and primer 27 (SEQ ID NO: 39).

A promoter-regulated Zeocin (tradename)-resistance gene (SEQ ID NO: 21), which was used for vector construction, was prepared by PCR using synthetic DNA as a template. A G418-resistance gene (SEQ ID NO: 22), which was used for vector construction, was prepared by PCR using synthetic DNA as a template. A green fluorescent protein gene (SEQ ID NO: 23), which was used for vector construction, was prepared by PCR using synthetic DNA as a template.

PCR was performed by using Prime STAR HS DNA Polymerase (Takara Bio Inc.) etc. under the reaction conditions described in the instructions included. The chromosome DNA was prepared from the *Komagataella pastoris* strain ATCC76273 using Kaneka Easy DNA Extraction Kit Version 2 (Kaneka Corporation) etc. under the conditions described therein.

### <Production Example 2: Construction of vector for inactivating the gene comprising the nucleotide sequence as shown in SEQ ID NO: 25 (DNL4 gene)>

A nucleic acid fragment in which *Pst*I recognition sequence and *Bam*HI recognition sequence were added to the ends of a DNL4 promoter (SEQ ID NO: 1) was prepared by PCR using primer 1 (SEQ ID NO: 7) and primer 2 (SEQ ID NO: 8). A nucleic acid fragment in which *Bam*HI recognition sequence and *Kpn*I recognition sequence were added to the ends of a DNL4 terminator (SEQ ID NO: 2) was prepared by PCR using primer 3 (SEQ ID NO: 9) and primer 4 (SEQ ID NO: 10). The nucleic acid fragment prepared from the DNL4 promoter was treated with *Pst*I and *Bam*HI*,* the nucleic acid fragment prepared from the DNL4 terminator was treated with *Bam*HI and *Kpn*I*,* and the resultants were inserted into a site between *Pst*I and *Kpn*I of pUC19 (Code No. 3219, Takara Bio Inc.). Thus, pUC-Pdnl4Tdnl4 was constructed.

Subsequently, a nucleic acid fragment in which *Kpn*I recognition sequences were added to the both sides of the promoter-regulated ADE1 gene sequence (SEQ ID NO: 3) was prepared by PCR using primer 5 (SEQ ID NO: 11) and primer 6 (SEQ ID NO: 12), treated with *Kpn*I*,* and inserted into a *Kpn*I site of pUC-Pdnl4Tdnl4. Thus, pUC-Pdnl4Tdnl4ADE1 was constructed.

This vector comprises, as homologous regions, a 1,000-bp promoter region and a 1,007-bp terminator region of the gene (DNL4 gene) comprising the nucleotide sequence as shown in SEQ ID NO: 25 encoding the amino acid sequence (Accession No. CCA39424) as shown in SEQ ID NO: 24, which is DNL4 of the *Komagataella pastoris* strain ATCC76273, and a promoter-regulated ADE1 gene added thereto. As described in the examples below, this vector is designed to inactivate the gene (DNL4 gene) comprising the nucleotide sequence as shown in SEQ ID NO: 25 by transforming the vector into the host methanol-utilizing yeast strain.

### <Production Example 3: Construction of the autonomously replicating vector expressing the green fluorescent protein>

A nucleic acid fragment in which *Hind*III*-Xba*I*-Not*I*-Asc*I*-Sfi*I*-Pac*I*-Asi*SI*-Sfi*I recognition sequence and *Eco*RI recognition sequence were added to the ends of the promoter-regulated Zeocin (tradename)-resistance gene (SEQ ID NO: 21) was prepared by PCR using primer 15 (SEQ ID NO: 26) and primer 16 (SEQ ID NO: 27), treated with *Hin*dIII and *EcoRI,* and inserted into a site between *Hin*dIII and *Eco*RI of pUC19. Thus, pUC-Zeo was constructed.

Subsequently, a nucleic acid fragment in which *Asc*I recognition sequence and *Spe*I recognition sequence were added to the ends of the GAP promoter (SEQ ID NO: 4) was prepared by PCR using primer 7 (SEQ ID NO: 13) and primer 8 (SEQ ID NO: 14). A nucleic acid fragment in which *Spe*I recognition sequence and *Xho*I recognition sequence were added to the ends of the green fluorescent protein gene (SEQ ID NO: 23) was prepared by PCR using primer 17 (SEQ ID NO: 28) and primer 18 (SEQ ID NO: 29). A nucleic acid fragment in which *Xho*I recognition sequence and *Pac*I recognition sequence were added to the ends of GAP1 terminator (SEQ ID NO: 5) was prepared by PCR using primer 9 (SEQ ID NO: 15) and primer 10 (SEQ ID NO: 16). The GAP promoter was treated with *Asc*I and *Spe*I*,* the green fluorescent protein gene was treated with *Spe*I and *Xho*I*,* and the GAP1 terminator was treated with *Xho*I and *Pac*I*.* The resultants were inserted into a site between *Asc*I and *Pac*I of pUC-Zeo. Thus, pUC-PgapGFPTgap1Zeo was constructed.

Subsequently, a region approximately a half of the centromeric DNA sequence (SEQ ID NO: 6) (i.e., LOR_CC) was prepared by PCR using primer 11 (SEQ ID NO: 17) and primer 12 (SEQ ID NO: 18). A remaining region of an approximately half of the centromeric DNA sequence (i.e., CC_ROR) was prepared by PCR using primer 13 (SEQ ID NO: 19) and primer 14 (SEQ ID NO: 20). LOR_CC was treated with *Not*I and *Pst*I*,* CC ROR was treated with *Pst*I and *Xba*I*,* and the resultants were inserted into a site between *Xba*I and *Not*I of pUC-PgapGFPTgap1Zeo. Thus, pUC-Cen2PgapGFPTgap1Zeo was constructed.

This vector comprising a centromeric DNA sequence including the autonomously replicating sequence (ARS) of *Komagataella pastoris* autonomously replicates. In this vector, the green fluorescent protein is regulated by the GAP promoter. A transformant transfromed with this vector has resistance to Zeocin (tradename).

PARS1 (SEQ ID NO: 37) was prepared by PCR using primer 26 (SEQ ID NO: 38) and primer 27 (SEQ ID NO: 39), treated with *Not*I*,* and inserted into the *Not*I site of pUC-PgapGFPTgap1Zeo. Thus, pUC-PARS1PgapGFPTgap1Zeo was constructed.

This vector comprising PARS1, which is ARS of *Komagataella pastoris,* autonomously replicates. In this vector, the green fluorescent protein is regulated by the GAP promoter. A transformant transfromed with this vector has resistance to Zeocin (tradename).

### <Production Example 4: Construction of the G418-resistance gene vector>

A nucleic acid fragment of the G418-resistance gene (SEQ ID NO: 22) was prepared by PCR using primer 19 (SEQ ID NO: 30) and primer 20 (SEQ ID NO: 31). Thus, 0_G418_0 was constructed.

A nucleic acid fragment of the G418-resistance gene (SEQ ID NO: 22) was prepared by PCR using primer 21 (SEQ ID NO: 32) and primer 22 (SEQ ID NO: 33). Thus, 30_G418_30 was constructed. This vector is designed to comprise, at its end, a 30-bp nucleotide sequence exhibiting 100% sequence identity to the GAP promoter and the GAP1 terminator.

A nucleic acid fragment of the G418-resistance gene (SEQ ID NO: 22) was prepared by PCR using primer 23 (SEQ ID NO: 34) and primer 24 (SEQ ID NO: 35). Thus, 60_G418_60 was constructed. This vector is designed to comprise, at its end, a 60-bp nucleotide sequence exhibiting 100% sequence identity to the GAP promoter and the GAP1 terminator.

### <Production Example 5: Construction of an autonomously replicating vector having a multiple cloning site>

A nucleic acid fragment in which AOX1 promoter (SEQ ID NO: 41) and GAP1 terminator (SEQ ID NO: 5) were added to the ends of the multiple cloning site *(Acc*65I*-Avr*II-*Eco*RV*-Mlu*I*-Bsr*GI) (SEQ ID NO: 40) was prepared by PCR using synthetic DNA as a template, primer 28 (SEQ ID NO: 42), and primer 29 (SEQ ID NO: 43). The nucleic acid fragment was treated with *Asc*I and *Pac*I and inserted into a site between *Asc*I and *Pac*I of pUC-Cen2PgapGFPTgap1Zeo constructed in Production Example 3. Thus, pUC-Cen2Paox1MCSTgap1Zeo was constructed. This vector comprising a centromeric DNA sequence including the autonomously replicating sequence (ARS) of *Komagataella pastoris* autonomously replicate. This vector fuether comprises a multiple cloning site (*Acc*65I*-Avr*II-*Eco*RV*-Mlu*I*-Bsr*GI) downstream of the AOX1 promoter and the GAP1 terminator downstream of the multiple cloning site. A transformant transformed with this vector has resistance to Zeocin (tradename).

### <Production Example 6: Construction of vectors>

A nucleic acid fragment comprising positions 855449 to 856477 of Chromosome No. 6 (AECK01000006) was prepared by PCR using, as a template, a mixture of chromosome DNA from the *Ogataea angusta* strain NCYC495 (the nucleotide sequences thereof are described in EMBL (The European Molecular Biology Laboratory) Accession Nos. AECK01000001 to AECK01000007), primer 30 (SEQ ID NO: 44), and primer 31(SEQ ID NO: 45). Thus, 1,106-bp Fragment 1 (SEQ ID NO: 46) was constructed.

Also, a nucleic acid fragment comprising positions 856357 to 858859 of Chromosome No.6 (AECK01000006) was prepared by PCR using primer 32 (SEQ ID NO: 47) and primer 33 (SEQ ID NO: 48). Thus, 2,503-bp Fragment 2 (SEQ ID NO: 49) was constructed.

Also, a nucleic acid fragment comprising positions 858744 to 861310 of Chromosome No.6 (AECK01000006) was prepared by PCR using primer 34 (SEQ ID NO: 50) and primer 35 (SEQ ID NO: 51). Thus, 2,567-bp Fragment 3 (SEQ ID NO: 52) was constructed.

Also, a nucleic acid fragment comprising positions 861181 to 863668 of Chromosome No.6 (AECK01000006) was prepared by PCR using primer 36 (SEQ ID NO: 53) and primer 37 (SEQ ID NO: 54). Thus, 2,488-bp Fragment 4 (SEQ ID NO: 55) was constructed.

Also, a nucleic acid fragment comprising positions 863538 to 866113 of Chromosome No.6 (AECK01000006) was prepared by PCR using primer 38 (SEQ ID NO: 56) and primer 39 (SEQ ID NO: 57). Thus, 2,624-bp Fragment 5 (SEQ ID NO: 58) was constructed.

A nucleic acid fragment comprising a nucleotide sequence of positions 1 to 195 of the green fluorescent protein gene to which the GAP promoter (SEQ ID NO: 4) was ligated was prepared by PCR using synthetic DNA as a template, primer 40 (SEQ ID NO: 59), and primer 41 (SEQ ID NO: 60). Thus, 735-bp PgapGFP1 (SEQ ID NO: 61) was constructed.

A nucleic acid fragment comprising a nucleotide sequence of nucleotides 101 to 720 of the green fluorescent protein gene was prepared by PCR using synthetic DNA as a template, primer 42 (SEQ ID NO: 62), and primer 43 (SEQ ID NO: 63). Thus, 696-bp GFP2 (SEQ ID NO: 64) was constructed.
Fragment 1 is designed to comprise, at the upper end, a 77-bp nucleotide sequence exhibiting 100% sequence identity to the AOX1 promoter and the multiple cloning site and, at the lower end, a 121-bp nucleotide sequence exhibiting 100% sequence identity to Fragment 2.
Fragment 2 is designed to comprise, at the upper end, a 121-bp nucleotide sequence exhibiting 100% sequence identity to Fragment 1 and, at the lower end, a 116-bp nucleotide sequence exhibiting 100% sequence identity to Fragment 3.
Fragment 3 is designed to comprise, at the upper end, a 116-bp nucleotide sequence exhibiting 100% sequence identity to Fragment 2 and, at the lower end, a 130-bp nucleotide sequence exhibiting 100% sequence identity to Fragment 4.
Fragment 4 is designed to comprise, at the upper end, a 130-bp nucleotide sequence exhibiting 100% sequence identity to Fragment 3 and, at the lower end, a 131-bp nucleotide sequence exhibiting 100% sequence identity to Fragment 5.
Fragment 5 is designed to comprise, at the upper end, a 131-bp nucleotide sequence exhibiting 100% sequence identity to Fragment 4 and, at the lower end, a 95-bp nucleotide sequence exhibiting 100% sequence identity to PgapGFP1.

PgapGFP1 is designed to comprise, at the upper end, a 95-bp nucleotide sequence exhibiting 100% sequence identity to Fragment 5 and, at the lower end, a 95-bp nucleotide sequence exhibiting 100% sequence identity to GFP2.

GFP2 is designed to comprise, at the upper end, a 95-bp nucleotide sequence exhibiting 100% sequence identity to PgapGFP1 and, at the lower end, a 76-bp nucleotide sequence exhibiting 100% sequence identity to the multiple cloning site and the GAP1 terminator.

**[Table 1]**

| Vector | Nucleotide sequence | Primer sequence (Fw) used for amplification of nucleic acid fragment | Primer sequence (Re) used for amplification of nucleic acid fragment | Position of Chromosome 6 (AECK01000006) | Length |
|---|---|---|---|---|---|
| Fragment 1 | SEQ ID NO: 46 | Primer 3 (SEQ ID NO: 44) | Primer 31 (SEQ ID NO: 45) | 855449-856477 | 1,106 bp |
| Fragment 2 | SEQ ID NO: 49 | Primer 32 (SEQ ID NO: 47) | Primer 33 (SEQ ID NO: 48) | 856357-858859 | 2,503 bp |
| Fragment 3 | SEQ ID NO: 52 | Primer 34 (SEQ ID NO: 50) | Primer 35 (SEQ ID NO: 51) | 858744-861310 | 2,567 bp |
| Fragment 4 | SEQ ID NO: 55 | Primer 36 (SEQ ID NO: 53) | Primer 37 (SEQ ID NO: 54) | 861181-863668 | 2,488 bp |
| Fragment 5 | SEQ ID NO: 58 | Primer 38 (SEQ ID NO: 56) | Primer 39 (SEQ ID NO: 57) | 863538-866113 | 2,624 bp |
| PgapGFP1 | SEQ ID NO: 61 | Primer 40 (SEQ ID NO: 59) | Primer 41 (SEQ ID NO: 60) | - | 735 bp |
| GFP2 | SEQ ID NO: 64 | Primer 42 (SEQ ID NO: 62) | Primer 43 (SEQ ID NO: 63) | - | 696 bp |

### <Example 1: Generation of transformed yeast in which the gene comprising the nucleotide sequence as shown in SEQ ID NO: 25 (DNL4 gene) is inactivated>

With the use of the vector for inactivating the gene comprising the nucleotide sequence as shown in SEQ ID NO: 25 (DNL4 gene) constructed in Production Example 2 (pUC-Pdnl4Tdnl4ADE1), a host methanol-utilizing yeast strain was transformed in the manner described below.

An adenine auxotrophic strain derived from the *Komagataella pastoris* strain ATCC76273 was inoculated in 3 ml of YPD medium (1% yeast extract bacto (Becton, Dickinson and Company), 2% hipolypeptone (Nihon Pharmaceutical Co., Ltd.), and 2% glucose) and shake-cultured overnight at 30°C to obtain a preculture suspension. The preculture suspension thus obtained (500 µl) was inoculated in 50 ml of YPD medium and shake-cultured up to an OD600 of 1 to 1.5. Thereafter, the yeast culture was harvested (3000× g, 10 minutes, 20°C) and resuspended in 10 ml of 50 mM potassium phosphate buffer (pH 7.5) supplemented with 250 µl of 1 M DTT (final concentration: 25 mM).

After the suspension was subjected to incubation for 15 minutes at 30°C, the yeast culture was harvested (3000× g, 10 minutes, 20°C) and washed with 50 ml of STM buffer precooled in ice (270 mM sucrose, 10 mM Tris-HCl, 1 mM magnesium chloride, pH 7.5). The culture was harvested from the washing solution (3000× g, 10 minutes, 4°C) and washed again with 25 ml of STM buffer, followed by harvesting (3000× g, 10 minutes, 4°C). In the end, the obtained yeast culture was suspended in 250 µl of the ice-cold STM buffer and the resulting suspension was designated as a competent cell suspension.

With the use of the vector for inactivating the gene comprising the nucleotide sequence as shown in SEQ ID NO: 25 (DNL4 gene) constructed in Production Example 2 (pUC-Pdnl4Tdnl4ADE1), *E. coli* was transformed, the transformant was cultured in 5 ml of ampicillin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Pdnl4Tdnl4ADE1 was obtained from the cultured cells using FastGene Plasmid Mini Kit (NIPPON Genetics Co., Ltd.). This plasmid was linearized via *Bgl*II treatment targeting a *Bgl*II recognition sequence within the DNL4 terminator.

The competent cell suspension obtained above (60 µl) was mixed with 3 µl of a solution of the linearized pUC-Pdnl4Tdnl4ADE1, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C) and suspended in 1 ml of YNB medium (0.67% Yeast Nitrogen Base Without Amino Acids (Becton, Dickinson and Company). Thereafter, the yeast culture was harvested again (3000× g, 5 minutes, 20°C), resuspended in an adequate amount of YNB medium, and then spread on a selective YNB agar plate (0.67% Yeast Nitrogen Base Without Amino Acids (Becton, Dickinson and Company), 1.5% agarose, and 2% glucose). A strain that grew in static culture for 3 days at 30°C was selected.

With the use of the selected strain, homologous recombination was performed to delete the gene comprising the nucleotide sequence as shown in SEQ ID NO: 25 (DNL4 gene) and the promoter-regulated ADE1 gene. Thus, the Δdnl4WΔade1 strain was obtained.

Subsequently, the ADE1 gene was introduced into the HIS4 gene region of the Δdnl4Δade1 strain to obtain the Δdnl4Δhis4 strain. This strain is a histidine auxotrophic transformed yeast strain in which the gene comprising the nucleotide sequence as shown in SEQ ID NO: 25 (DNL4 gene) has been inactivated.

### <Comparative Example 1: Generation of transformed yeast, calculation of transformation efficiency, and confirmation of self-circularization>

With the use of the autonomously replicating vector expressing the green fluorescent protein constructed in Production Example 3 (pUC-Cen2PgapGFPTgap1Zeo), the wild-type strain of the *Komagataella pastoris* strain ATCC76273 was transformed in the manner described below.

The wild-type strain was inoculated in 3 ml of YPD medium (1% yeast extract bacto (Becton, Dickinson and Company), 2% hipolypeptone (Nihon Pharmaceutical Co., Ltd.), and 2% glucose) and shake-cultured overnight at 30°C to obtain a preculture suspension. The preculture suspension thus obtained (500 µl) was inoculated in 50 ml of YPD medium and shake-cultured up to an OD600 of 1 to 1.5. Thereafter, the yeast culture was harvested (3000× g, 10 minutes, 20°C) and resuspended in 10 ml of 50 mM potassium phosphate buffer (pH 7.5) supplemented with 250 µl of 1M DTT (final concentration: 25 mM).

After the suspension was subjected to incubation for 15 minutes at 30°C, the yeast culture was harvested (3000× g, 10 minutes, 20°C) and washed with 50 ml of STM buffer precooled in ice (270 mM sucrose, 10 mM Tris-HCl, 1 mM magnesium chloride, pH 7.5). The culture was harvested from the washing solution (3000× g, 10 minutes, 4°C) and washed again with 25 ml of STM buffer, followed by harvesting (3000× g, 10 minutes, 4°C). In the end, the obtained yeast culture was suspended in 250 µl of the ice-cold STM buffer and the resulting suspension was designated as a competent cell suspension.

With the use of the autonomously replicating vector expressing the green fluorescent protein constructed in Production Example 3 (pUC-Cen2PgapGFPTgap1Zeo), *E. coli* was transformed, the transformant was cultured in 5 ml of ampicilin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Cen2PgapGFPTgap1Zeo was obtained from the cultured cells using FastGene Plasmid Mini Kit (NIPPON Genetics Co., Ltd.). This plasmid was linearized via *Bsr*GI treatment targeting a *Bsr*GI recognition sequence within the green fluorescent protein gene.

The competent cell suspension obtained above (60 µl) was mixed with 3 µl of a solution of the linearized pUC-Cen2PgapGFPTgap1Zeo, or the competent cell suspension obtained above (60 µl) was mixed with 3 µl of a solution of the pUC-Cen2PgapGFPTgap1Zeo that was not subjected to *BsrGI* treatment, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and 950 µl of the supernatant was discarded. The yeast culture was resuspended in a remaining solution and then spread on a selective YPD Zeocin (tradename) agar plate (1% yeast extract (Becton, Dickinson and Company), 2% hipolypeptone (Nihon Pharmaceutical Co., Ltd.), 2% glucose, 1.5% agarose, and 0.01% Zeocin (tradename) (Thermo Fisher Scientific, Inc.)). A strain that grew in static culture for 3 days at 30°C was selected, and transformation efficiency was calculated (the number of colonies per µg of vector; cfu/µg) (Table 2).

**[Table 2]**

| Condition | Host | Vector | Transformation efficiency (cfu/µg) |
|---|---|---|---|
| 1 | Wild-type | pUC-Cen2PgapGFPTgap1Zeo (treated with BsrGI) | 8.1×10³ |
| 2 | Wild-type | pUC-Cen2PgapGFPTgap1Zeo (not treated with BsrGI) | 7.1×10³ |

As a result, transformation efficiency of Condition 1 attained with the use of pUC-Cen2PgapGFPTgap1Zeo linearized via *BsrGI* treatment was found to be equivalent to transformation efficiency of Condition 2 attained with the use of pUC-Cen2PgapGFPTgap1Zeo that was not subjected to *Bsr*GI treatment. This indicates that self-circularization of the linearized pUC-Cen2PgapGFPTgap1Zeo has occurred because the wild-type strain has non-homologous end joining activity.

The 10 transformed yeast strains obtained under Condition 1 were inoculated on a selective YPD Zeocin (tradename) agar plate and subjected to static culture at 30°C for 1 day. A plasmid solution was obtained from the grown strains using Easy Yeast Plasmid Isolation Kit (Clontech Laboratories, Inc.) and then introduced into *E. coli* HST08 competent cells (Takara Bio Inc.). The *E. coli* transformants were cultured to amplify the plasmid, and then the plasmids were subjected to electrophoresis, sequence analysis, and other analytical techniques. As a result, it was found that self-circularization of pUC-Cen2PgapGFPTgap1Zeo linearized via *Bsr*GI treatment had occurred in all samples.

### <Example 2: Generation of transformed yeast and calculation of transformation efficiency>

With the use of the autonomously replicating vector expressing the green fluorescent protein constructed in Production Example 3 (pUC-Cen2PgapGFPTgap1Zeo), the histidine auxotrophic transformed yeast strain Δdnl4Δhis4 constructed in Example 1 in which the gene comprising the nucleotide sequence as shown in SEQ ID NO: 25 (DNL4 gene) had been inactivated was transformed.

The Δdnl4Δhis4 strain was inoculated in 3 ml of YPD medium (1% yeast extract bacto (Becton, Dickinson and Company), 2% hipolypeptone (Nihon Pharmaceutical Co., Ltd.), and 2% glucose) and shake-cultured overnight at 30°C to obtain a preculture suspension. The preculture suspension thus obtained (500 µl) was inoculated in 50 ml of YPD medium and shake-cultured up to an OD600 of 1 to 1.5. Thereafter, the yeast culture was harvested (3000× g, 10 minutes, 20°C) and resuspended in 10 ml of 50 mM potassium phosphate buffer (pH 7.5) supplemented with 250 µl of 1M DTT (final concentration: 25 mM).

After the suspension was subjected to incubation for 15 minutes at 30°C, the yeast culture was harvested (3000× g, 10 minutes, 20°C) and washed with 50 ml of STM buffer precooled in ice (270 mM sucrose, 10 mM Tris-HCl, 1 mM magnesium chloride, pH 7.5). The culture was harvested from the washing solution (3000× g, 10 minutes, 4°C) and washed again with 25 ml of STM buffer, followed by harvesting (3000× g, 10 minutes, 4°C). In the end, the obtained yeast culture was suspended in 250 µl of the ice-cold STM buffer and the resulting suspension was designated as a competent cell suspension.

With the use of the autonomously replicating vector expressing the green fluorescent protein constructed in Production Example 3 (pUC-Cen2PgapGFPTgap1Zeo), *E. coli* was transformed, the transformant was cultured in 5 ml of ampicilin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Cen2PgapGFPTgap1Zeo was obtained from the cultured cells using FastGene Plasmid Mini Kit (NIPPON Genetics Co., Ltd.). This plasmid was linearized via *Bsr*GI treatment targeting a *Bsr*GI recognition sequence within the green fluorescent protein gene.

The competent cell suspension obtained above (60 µl) was mixed with 3 µl of a solution of the linearized pUC-Cen2PgapGFPTgap1Zeo, or the competent cell suspension obtained above (60 µl) was mixed with 3 µl of a solution of the pUC-Cen2PgapGFPTgap1Zeo that was not subjected to *Bsr*GI treatment, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and 950 µl of the supernatant was discarded. The yeast culture was resuspended in a remaining solution, and the suspension was spread on a selective YPD Zeocin (tradename) agar plate (1% yeast extract (Becton, Dickinson and Company), 2% hipolypeptone (Nihon Pharmaceutical Co., Ltd.), 2% glucose, 1.5% agarose, and 0.01% Zeocin (tradename) (Thermo Fisher Scientific, Inc.)). A strain that grew in static culture for 3 days at 30°C was selected, and transformation efficiency was calculated (the number of colonies per µg of vector; cfu/µg) (Table 3).

**[Table 3]**

| Condition | Host | Vector | Transformation efficiency (cfu/µg) |
|---|---|---|---|
| 3 | Δdnl4Δhis4 | pUC-Cen2PgapGFPTgap1Zeo (treated with BsrGI) | 0.0 |
| 4 | Δdnl4Δhis4 | pUC-Cen2PgapGFPTgap1Zeo (not treated with BsrGI) | 2.4×10³ |

As a result, transformation efficiency of Condition attained 3 with the use of pUC-Cen2PgapGFPTgap1Zeo linearized via *Bsr*GI treatment was found to have decreased to a significant extent, compared with transformation efficiency of Condition 4 attained with the use of pUC-Cen2PgapGFPTgap1Zeo that was not subjected to *Bsr*GI treatment, and no colonies were generated. This indicates that the Δdnl4Δhis4 strain has lost non-homologous end joining activity and self-circularization of the linearized pUC-Cen2PgapGFPTgap1Zeo is completely suppressed upon inactivation of the gene comprising the nucleotide sequence as shown in SEQ ID NO: 25 (DNL4 gene).

### <Comparative Example 2: Generation of transformed yeast, calculation of transformation efficiency, and calculation of assembly efficiency>

In order to assemble vectors, the autonomously replicating vector expressing the green fluorescent protein constructed in Production Example 3 (pUC-Cen2PgapGFPTgap1Zeo) and the G418-resistance gene vector constructed in Production Example 4 (60_G418_60) were used to transform the wild-type strain of the *Komagataella pastoris* strain ATCC76273 in the manner described below.

The wild-type strain was inoculated in 3 ml of YPD medium (1% yeast extract bacto (Becton, Dickinson and Company), 2% hipolypeptone (Nihon Pharmaceutical Co., Ltd.), and 2% glucose) and shake-cultured overnight at 30°C to obtain a preculture suspension. The preculture suspension thus obtained (500 µl) was inoculated in 50 ml of YPD medium and shake-cultured up to an OD600 of 1 to 1.5. Thereafter, the yeast culture was harvested (3000× g, 10 minutes, 20°C) and resuspended in 10 ml of 50 mM potassium phosphate buffer (pH 7.5) supplemented with 250 µl of 1M DTT (final concentration: 25 mM).

After the suspension was subjected to incubation for 15 minutes at 30°C, the yeast culture was harvested (3000× g, 10 minutes, 20°C) and washed with 50 ml of STM buffer precooled in ice (270 mM sucrose, 10 mM Tris-HCl, 1 mM magnesium chloride, pH 7.5). The culture was harvested from the washing solution (3000× g, 10 minutes, 4°C) and washed again with 25 ml of STM buffer, followed by harvesting (3000× g, 10 minutes, 4°C). In the end, the obtained yeast culture was suspended in 250 µl of the ice-cold STM buffer and the resulting suspension was designated as a competent cell suspension.

With the use of the autonomously replicating vector expressing the green fluorescent protein constructed in Production Example 3 (pUC-Cen2PgapGFPTgap1Zeo), *E. coli* was transformed, the transformant was cultured in 5 ml of ampicilin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Cen2PgapGFPTgap1Zeo was obtained from the cultured cells using FastGene Plasmid Mini Kit (NIPPON Genetics Co., Ltd.). This plasmid was linearized via *Bsr*GI treatment targeting a *Bsr*GI recognition sequence within the green fluorescent protein gene.

The competent cell suspension obtained above (60 µl), a solution of the linearized pUC-Cen2PgapGFPTgap1Zeo (0.015 pmol), and the G418-resistance gene vector 60_G418_60 (0.75 pmol) were mixed with each other, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and 950 µl of the supernatant was discarded. The yeast culture was resuspended in a remaining solution and spread on a selective YPD Zeocin (tradename) agar plate (1% yeast extract (Becton, Dickinson and Company), 2% hipolypeptone (Nihon Pharmaceutical Co., Ltd.), 2% glucose, 1.5% agarose, and 0.01% Zeocin (tradename) (Thermo Fisher Scientific, Inc.)). A strain that grew in static culture for 3 days at 30°C was selected, and transformation efficiency was calculated (the number of colonies per µg of vector; cfu/µg) (Table 4, Condition 5).

The 20 transformed yeast strains obtained under Condition 5 were inoculated on a selective YPD Zeocin (tradename) agar plate and subjected to static culture at 30°C for 1 day. In order to confirm that vectors had been assembled, chromosome DNA and a plasmid solution were obtained from the grown strains using Kaneka Easy DNA Extraction Kit Version 2 (Kaneka Corporation) and subjected to PCR with the use thereof as a template, primer 19 (SEQ ID NO: 30) as a forward primer for the G418-resistance gene, and primer 25 (SEQ ID NO: 36) as a reverse primer at a position 156 bp away from the origin of the GAP1 terminator. The PCR product was electrophoresed, and assembly efficiency was calculated based on the number of samples in which a band of the size of interest (951 bp) had developed (Table 4, Condition 5).

### <Example 3: Generation of transformed yeast, calculation of transformation efficiency, and calculation of assembly efficiency>

In order to assemble vectors, the autonomously replicating vectors expressing the green fluorescent protein constructed in Production Example 3 (pUC-Cen2PgapGFPTgap1Zeo and pUC-PARS1PgapGFPTgap1Zeo) and the G418-resistance gene vectors constructed in Production Example 4 (0_G418_0, 30_G418_30, and 60_G418_60) were used to transform the histidine auxotrophic transformed yeast strain Δdnl4Δhis4 constructed in Example 1 in which the gene comprising the nucleotide sequence as shown in SEQ ID NO: 25 (DNL4 gene) had been inactivated in the manner described below.

The Δdnl4Δhis4 strain was inoculated in 3 ml of YPD medium (1% yeast extract bacto (Becton, Dickinson and Company), 2% hipolypeptone (Nihon Pharmaceutical Co., Ltd.), and 2% glucose) and shake-cultured overnight at 30°C to obtain a preculture suspension. The preculture suspension thus obtained (500 µl) was inoculated in 50 ml of YPD medium and shake-cultured up to an OD600 of 1 to 1.5. Thereafter, the yeast culture was harvested (3000× g, 10 minutes, 20°C) and resuspended in 10 ml of 50 mM potassium phosphate buffer (pH 7.5) supplemented with 250 µl of 1M DTT (final concentration: 25 mM).

After the suspension was subjected to incubation for 15 minutes at 30°C, the yeast culture was harvested (3000× g, 10 minutes, 20°C) and washed with 50 ml of STM buffer precooled in ice (270 mM sucrose, 10 mM Tris-HCl, 1 mM magnesium chloride, pH 7.5). The culture was harvested from the washing solution (3000× g, 10 minutes, 4°C) and washed again with 25 ml of STM buffer, followed by harvesting (3000× g, 10 minutes, 4°C). In the end, the obtained yeast culture was suspended in 250 µl of the ice-cold STM buffer and the resulting suspension was designated as a competent cell suspension.

With the use of the autonomously replicating vector expressing the green fluorescent protein constructed in Production Example 3 (pUC-Cen2PgapGFPTgap1Zeo or pUC-PARS1PgapGFPTgap1Zeo), *E. coli* was transformed, the transformant was cultured in 5 ml of ampicilin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Cen2PgapGFPTgap1Zeo or pUC-PARS1PgapGFPTgap1Zeo was obtained from the cultured cells using FastGene Plasmid Mini Kit (NIPPON Genetics Co., Ltd.). This plasmid was linearized via *Bsr*GI treatment targeting a *Bsr*GI recognition sequence within the green fluorescent protein gene.

The competent cell suspension obtained above (60 µl), a solution of linearized pUC-Cen2PgapGFPTgap1Zeo (0.015 pmol) or a solution of linearized pUC-PARSlPgapGFPTgaplZeo (0.015 pmol), and the G418-resistance gene vector 0_G418_0, 30_G418_30, or 60_G418_60 (0.15 pmol or 0.75 pmol) were mixed with each other, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and 950 µl of the supernatant was discarded. The yeast culture was resuspended in a remaining solution and spread on a selective YPD Zeocin (tradename) agar plate (1% yeast extract (Becton, Dickinson and Company), 2% hipolypeptone (Nihon Pharmaceutical Co., Ltd.), 2% glucose, 1.5% agarose, and 0.01% Zeocin (tradename) (Thermo Fisher Scientific, Inc.)). A strain that grew in static culture for 3 days at 30°C was selected, and transformation efficiency was calculated (the number of colonies per µg of vector; cfu/µg) (Table 4, Conditions 6 to 12).

The 10 transformed yeast strains obtained under Conditions 8 and 9, the 20 transformed yeast strains obtained under Conditions 10 and 11, and the 10 transformed yeast strains obtained under Condition 12 were inoculated on a selective YPD Zeocin (tradename) agar plate and subjected to static culture at 30°C for 1 day. In order to confirm that vectors had been assembled, chromosome DNA and a plasmid solution were obtained from the grown strains using Kaneka Easy DNA Extraction Kit Version 2 (Kaneka Corporation) and subjected to PCR with the use thereof as a template, primer 19 (SEQ ID NO: 30) as a forward primer for the G418-resistance gene, and primer 25 (SEQ ID NO: 36) as a reverse primer at a position 156 bp away from the origin of the GAP1 terminator. The PCR product was electrophoresed, and assembly efficiency was calculated based on the number of samples in which a band of the size of interest (951 bp) had developed (Table 4, Conditions 8 to 12).

**[Table 4]**

| Condition | Host | Vector (1) | Vector (2) | Molar ratio (Vector (1):Vector (2)) | Transformation efficiency (cfu/µg) | Assembly efficiency (%) |
|---|---|---|---|---|---|---|
| 5 | Wild-type | pUC-Cen2PgapGFPTgap1Zeo (treated with BsrGI) | 60_G418_60 | 1:50 | 1.1×10⁴ | 20 |
| 6 | Δdnl4Δhis4 | pUC-Cen2PgapGFPTgap1Zeo (treated with BsrGI) | 0_G418_0 | 1:10 | 0 | - |
| 7 | Δdnl4Δhis4 | pUC-Cen2PgapGFPTgap1Zeo (treated with BsrGI) | 0_G418_0 | 1:50 | 0 | - |
| 8 | Δdnl4Δhis4 | pUC-Cen2PgapGFPTgap1Zeo (treated with BsrGI) | 30_G418_30 | 1:10 | 50 | 80 |
| 9 | Δdnl4Δhis4 | pUC-Cen2PgapGFPTgap1Zeo (treated with BsrGI) | 30_G418_30 | 1:50 | 50 | 100 |
| 10 | Δdnl4Δhis4 | pUC-Cen2PgapGFPTgap1Zeo (treated with BsrGI) | 60_G418_60 | 1:10 | 2.4×10² | 95 |
| 11 | Δdnl4Δhis4 | pUC-Cen2PgapGFPTgap1Zeo (treated with BsrGI) | 60_G418_60 | 1:50 | 9.2×10³ | 100 |
| 12 | Δdnl4Δhis4 | pUC-PARS1PgapGFPTgap1Zeo (treated with BsrGI) | 60_G418_60 | 1:50 | 9.1×10³ | 100 |

As a result, the assembly efficiency of Conditions 5 attained with the use of pUC-Cen2PgapGFPTgap1Zeo linearized via *Bsr*GI treatment and the G418-resistance gene vector 60_G418_60 designed to comprise a 60-bp nucleotide sequence exhibiting 100% sequence identity to the GAP promoter and the GAP1 terminator was found to be as 20%. This indicates that 60_G418_60 is not efficiently assembled in the wild-type strain due to self-circularization of linearized pUC-Cen2PgapGFPTgap1Zeo because the wild-type strain has non-homologous end joining activity.

Condition 6 and Condition 7 are each prepared via transformation into the host Δdnl4Δhis4 strain with the use of pUC-Cen2PgapGFPTgap1Zeo linearized via *sr*GI treatment and the G418-resistance gene vector 0_G418_0 that is not designed to comprise a nucleotide sequence exhibiting sequence identity to the GAP promoter and the GAP1 terminator. As with the results shown in Table 3 in Example 3, self-circularization of linearized pUC-Cen2PgapGFPTgap1Zeo was suppressed.

Meanwhile, the assembly efficiency of Conditions 8 to 11 attained with the use of linearized pUC-Cen2PgapGFPTgap1Zeo and the G418-resistance gene vectors 30_G418_30 and 60_G418_60 was high. This indicates that the Δdnl4Δhis4 strain has lost non-homologous end joining activity upon inactivation of the gene comprising the nucleotide sequence as shown in SEQ ID NO: 25 (DNL4 gene), self-circularization of linearized pUC-Cen2PgapGFPTgap1Zeo has been completely suppressed, and the vectors are efficiently assembled in the yeast strains.

Assembly efficiency of Condition 12 attained with the use of linearized pUC-PARSlPgapGFPTgaplZeo and the G418-resistance gene vector 60_G418_60 was high. This indicates that vectors are assembled with each other efficiently in yeast regardless of a difference in ARS because the Δdnl4Δhis4 strain has lost non-homologous end joining activity upon inactivation of the gene comprising the nucleotide sequence as shown in SEQ ID NO: 25 (DNL4 gene) and self-circularization of linearized pUC-PARSlPgapGFPTgaplZeo has been completely suppressed.

A plasmid solution was obtained from the strain in which vector assembly was confirmed via PCR using primer 19 (SEQ ID NO: 30) and primer 25 (SEQ ID NO: 36) using Easy Yeast Plasmid Isolation Kit (Clontech Laboratories, Inc.) and introduced into *E. coli* HST08 competent cells (Takara Bio Inc.). The *E. coli* transformants were cultured to amplify plasmids, and the plasmids were subjected to sequence analysis. As a result, it was found that the green fluorescent protein gene of pUC-Cen2PgapGFPTgap1Zeo linearized via *BsrGI* treatment had been substituted with the G418-resistance gene in all samples.

### <Comparative Example 3: Generation of transformed yeast, calculation of transformation efficiency, and calculation of assembly efficiency>

In order to assemble vectors, the wild-type strain of the *Komagataella pastoris* strain ATCC76273 was transformed in the same manner as in Comparative Example 2 with the use of the autonomously replicating vector having a multiple cloning site constructed in Production Example 5 (pUC-Cen2Paox1MCSTgap1Zeo), Fragment 1, Fragment 2, Fragment 3, Fragment 4, Fragment 5, PgapGFP1, and GFP2 constructed in Production Example 6.

With the use of the autonomously replicating vector having a multiple cloning site constructed in Production Example 5 (pUC-Cen2Paox1MCSTgap1Zeo), *E. coli* was transformed, the transformant was cultured in 5 ml of ampicilin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Cen2Paox1MCSTgap1Zeo was obtained from the cultured cells using FastGene Plasmid Mini Kit (NIPPON Genetics Co., Ltd.). This plasmid was linearized via *Mlu*I treatment digesting a *Mlu*I recognition sequence within the multiple cloning site.

The competent cell suspension obtained in Comparative Example 2 (60 µl), a solution of the linearized pUC-Cen2Paox1MCSTgap1Zeo (0.015 pmol), a solution of Fragment 1 constructed in Production Example 6 (0.15 pmol), a solution of Fragment 2 constructed in Production Example 6 (0.15 pmol), a solution of Fragment 3 constructed in Production Example 6 (0.15 pmol), a solution of Fragment 4 constructed in Production Example 6 (0.15 pmol), a solution of Fragment 5 constructed in Production Example 6 (0.15 pmol), a solution of PgapGFP1 constructed in Production Example 6 (0.15 pmol), and a solution of GFP2 constructed in Production Example 6 (0.15 pmol) were mixed with each other, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and 950 µl of the supernatant was discarded. The yeast culture was resuspended in a remaining solution and spread on a selective YPD Zeocin (tradename) agar plate (1% yeast extract (Becton, Dickinson and Company), 2% hipolypeptone (Nihon Pharmaceutical Co., Ltd.), 2% glucose, 1.5% agarose, and 0.01% Zeocin (tradename) (Thermo Fisher Scientific, Inc.)). A strain that grew in static culture for 3 days at 30°C was selected, and transformation efficiency was calculated (the number of colonies per µg of vector; cfu/µg) (Table 5, Condition 13).

The 10 transformed yeast strains obtained under Condition 13 were inoculated on a selective YPD Zeocin (tradename) agar plate and subjected to static culture at 30°C for 1 day. In order to confirm that vectors had been assembled, chromosome DNA and a plasmid solution were obtained from the grown strains using Kaneka Easy DNA Extraction Kit Version 2 (Kaneka Corporation) and subjected to PCR with the use thereof as a template, primer 44 (SEQ ID NO: 65) as a forward primer at a position 920 bp away from the origin of the AOX1 promoter, and primer 25 (SEQ ID NO: 36) as a reverse primer at a position 156 bp away from the origin of the GAP1 terminator. The PCR product was electrophoresed, and assembly efficiency was calculated based on the number of samples in which a band of the size of interest (about 12 kbp) had developed (Table 5, Condition 13).

The fluorescence-emitting strains were selected from the 1,123 transformed yeast strains obtained under Condition 13, the number thereof was counted, and the proportion thereof was determined (Table 5, Condition 13).

### <Example 4: Generation of transformed yeast, calculation of transformation efficiency, and calculation of assembly efficiency>

In order to assemble vectors, the autonomously replicating vector having a multiple cloning site constructed in Production Example 5 (pUC-Cen2Paox1MCSTgap1Zeo), Fragment 1, Fragment 2, Fragment 3, Fragment 4, Fragment 5, PgapGFP1, and GFP2 constructed in Production Example 6 were used to transform the histidine auxotrophic transformed yeast strain Δdnl4Δhis4 constructed in Example 1 in which the gene comprising the nucleotide sequence as shown in SEQ ID NO: 25 (DNL4 gene) had been inactivated in the same manner as in Example 3.

With the use of the autonomously replicating vector having a multiple cloning site constructed in Production Example 5 (pUC-Cen2Paox1MCSTgap1Zeo), *E. coli* was transformed, the transformant was cultured in 5 ml of ampicilin-containing 2YT medium (1.6% tryptone bacto (Becton, Dickinson and Company), 1% yeast extract bacto (Becton, Dickinson and Company), 0.5% sodium chloride, and 0.01% ampicillin sodium (Wako Pure Chemical Corporation)), and pUC-Cen2Paox1MCSTgap1Zeo was obtained from the cultured cells using FastGene Plasmid Mini Kit (NIPPON Genetics Co., Ltd.). This plasmid was linearized via *Mlu*I treatment targeting a *Mlu*I recognition sequence within the multiple cloning site.

The competent cell suspension obtained in Example 3 (60 µl), a solution of the linearized pUC-Cen2Paox1MCSTgap1Zeo (0.015 pmol), a solution of Fragment 1 constructed in Production Example 6 (0.15 pmol), a solution of Fragment 2 constructed in Production Example 6 (0.15 pmol), a solution of Fragment 3 constructed in Production Example 6 (0.15 pmol), a solution of Fragment 4 constructed in Production Example 6 (0.15 pmol), a solution of Fragment 5 constructed in Production Example 6 (0.15 pmol), a solution of PgapGFP1 constructed in Production Example 6 (0.15 pmol), and a solution of GFP2 constructed in Production Example 6 (0.15 pmol) were mixed with each other, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and 950 µl of the supernatant was discarded. The yeast culture was resuspended in a remaining solution and spread on a selective YPD Zeocin (tradename) agar plate (1% yeast extract (Becton, Dickinson and Company), 2% hipolypeptone (Nihon Pharmaceutical Co., Ltd.), 2% glucose, 1.5% agarose, and 0.01% Zeocin (tradename) (Thermo Fisher Scientific, Inc.)). A strain that grew in static culture for 3 days at 30°C was selected, and transformation efficiency was calculated (the number of colonies per µg of vector; cfu/µg) (Table 5, Condition 14).

The 10 transformed yeast strains obtained under Condition 14 were inoculated on a selective YPD Zeocin (tradename) agar plate and subjected to static culture at 30°C for 1 day. In order to confirm that vectors had been assembled, chromosome DNA and a plasmid solution were obtained from the grown strains using Kaneka Easy DNA Extraction Kit Version 2 (Kaneka Corporation) and subjected to PCR with the use thereof as a template, primer 44 (SEQ ID NO: 65) as a forward primer at a position 920 bp away from the origin of the AOX1 promoter, and primer 25 (SEQ ID NO: 36) as a reverse primer at a position 156 bp away from the origin of the GAP1 terminator. The PCR product was electrophoresed, and assembly efficiency was calculated based on the number of samples in which a band of the size of interest (about 12 kbp) had developed (Table 5, Condition 14).

The fluorescence-emitting strains were selected from the 561 transformed yeast strains obtained under Condition 14, the number thereof was counted, and the proportion thereof was determined (Table 5, Condition 14).

**[Table 5]**

| Condition | Host | Vector (1) | Vector (2) | Molar ratio (Vector (1):Vector (2)) | Transformation efficiency (cfu/µg) | Assembly efficiency (%) | Proportion of fluorescence-emitting strains (%) |
|---|---|---|---|---|---|---|---|
| 13 | Wild-type | pUC-Cen2Paox1MCSTgap1Zeo (treated with Mlul) | Fragments 1 to 5, PgapGFP1, GFP2 | 1:10 | 9.7×10³ | 20 | 13 |
| 14 | Δdnl4Δhis4 | pUC-Cen2Paox1MCSTgap1Zeo (treated with Mlul) | Fragments 1 to 5, PgapGFP1, GFP2 | 1:10 | 4.8×10³ | 100 | 95 |

As a result, the assembly efficiency of Condition 13 attained with the use of pUC-Cen2Paox1MCSTgap1Zeo linearized via *Mlu*I treatment, Fragment 1, Fragment 2, Fragment 3, Fragment 4, Fragment 5, PgapGFP1, and GFP2 were found to be as low as 20%, and the proportion of fluorescence-emitting strains was found to be as low as 13%. This indicates that vectors are not efficiently assembled in yeast due to, for example, self-circularization of linearized pUC-Cen2Paox1MCSTgap1Zeo that had occurred because of non-homologous end joining activity of the wild-type strain.

Meanwhile, the assembly efficiency of Condition 14 attained with the use of pUC-Cen2Paox1MCSTgap1Zeo linearized via *Mlu*I treatment, Fragment 1, Fragment 2, Fragment 3, Fragment 4, Fragment 5, PgapGFP1, and GFP2 was as high as 100%, and the proportion of fluorescence-emitting strains was as high as 95%. This indicates that vectors are assembled with each other efficiently in yeast because the Δdnl4Δhis4 strain has lost non-homologous end joining activity upon inactivation of the gene comprising the nucleotide sequence as shown in SEQ ID NO: 25 (DNL4 gene) and self-circularization of linearized pUC-Cen2Paox1MCSTgap1Zeo has been completely suppressed.

A plasmid solution was obtained from the strain in which vector assembly was confirmed via PCR using primer 44 (SEQ ID NO: 65) and primer 25 (SEQ ID NO: 36) using Easy Yeast Plasmid Isolation Kit (Clontech Laboratories, Inc.) and introduced into *E. coli* HST08 competent cells (Takara Bio Inc.). The *E. coli* transformants were cultured to amplify plasmids, and the plasmids were subjected to sequence analysis. As a result, it was found that 7 nucleic acid fragments; i.e., Fragment 1, Fragment 2, Fragment 3, Fragment 4, Fragment 5, PgapGFP1, and GFP2, had been inserted in that order into the multiple cloning site of pUC-Cen2Paox1MCSTgap1Zeo linearized via *Mlu*I treatment in all samples.

On the basis of the results above, the present inventors found that self-circularization of a vector could be completely suppressed by inactivating a gene encoding a DNL4 protein associated with non-homologous end joining. The present inventors also found that vectors could be assembled efficiently in a yeast cell in which the gene is inactivated. Specifically, the present inventors succeeded in efficiently assembling vectors in yeast by a transformation technique comprising a step of introducing two or more types of vectors into a methanol-utilizing yeast strain in which the DNL4 gene associated with non-homologous end joining has been inactivated.

### <Production Example 7: Construction of vector for genome integration>

A nucleic acid fragment comprising the green fluorescent protein gene (SEQ ID NO: 23) to which the GAP promoter (SEQ ID NO: 4) has been ligated was prepared by PCR using synthetic DNA as a template, primer 45 (SEQ ID NO: 66), and primer 46 (SEQ ID NO: 67). Thus, 1,309-bp Fragment G1 (SEQ ID NO: 68) was constructed.

A nucleic acid fragment comprising a nucleotide sequence of nucleotides 1 to 238 of the CCA38473 terminator downstream of the AOX1 terminator was prepared by PCR using synthetic DNA as a template, primer 47 (SEQ ID NO: 69), and primer 48 (SEQ ID NO: 70). Thus, 584-bp Fragment G2 (SEQ ID NO: 71) was constructed.

A nucleic acid fragment comprising the promoter-regulated G418-resistance gene (SEQ ID NO: 22) and the nucleotide sequence of pUC19 downstream of the nucleotide sequence of nucleotides 239 to 600 of the CCA38473 terminator was prepared by PCR using synthetic DNA as a template, primer 49 (SEQ ID NO: 72), and primer 50 (SEQ ID NO: 73). Thus, 4,095-bp Fragment G3 (SEQ ID NO: 74) was constructed.

Fragment G1 is designed to comprise, at the upper end, a 50-bp nucleotide sequence exhibiting 100% sequence identity to the lower end of Fragment G3 and, at the lower end, a 46-bp nucleotide sequence exhibiting 100% sequence identity to the upper end of Fragment G2.

Fragment G2 is designed to comprise, at the upper end, a 46-bp nucleotide sequence exhibiting 100% sequence identity to the lower end of Fragment G1 and, at the lower end, a 238-bp nucleotide sequence exhibiting 100% sequence identity to the CCA38473 terminator of the genome.

Fragment G3 is designed to comprise, at the upper end, a 362-bp nucleotide sequence exhibiting 100% sequence identity to the CCA38473 terminator of the genome and, at the lower end, a 50-bp nucleotide sequence exhibiting 100% sequence identity to the upper end of Fragment G1.

Fig. 1 shows the correlation of the vectors.

### <Comparative Example 4: Generation of transformed yeast, calculation of transformation efficiency, and calculation of assembly efficiency>

In order to integrate the assembled vectors into the genome, Fragment G1, Fragment G2, and Fragment G3 constructed in Production Example 7 were used to transform the wild-type strain of the *Komagataella pastoris* strain ATCC76273 in the same manner as in Comparative Example 2.

The competent cell suspension obtained in Comparative Example 2 (60 µl), a solution of Fragment G1 constructed in Production Example 7 (0.3 pmol), a solution of Fragment G2 constructed in Production Example 7 (0.3 pmol), and a solution of Fragment G3 constructed in Production Example 7 (0.015 pmol) were mixed with each other, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and the supernatant was discarded. The yeast culture was resuspended in 1 ml of YNB solution (0.17% Yeast Nitrogen Base without Amino Acids and Ammonium Sulfate (Becton, Dickinson and Company) and 0.1% sodium glutamate (Nacalai Tesque Inc.)), the yeast culture was harvested (3000× g, 5 minutes, 20°C), and 950 µl of the supernatant was discarded. The yeast culture was resuspended in a remaining solution and spread on a selective SD G418 agar plate (0.17% Yeast Nitrogen Base without Amino Acids and Ammonium Sulfate (Becton, Dickinson and Company), 0.1% sodium glutamate (Nacalai Tesque Inc.), 2% glucose, 1.5% agarose, and 0.05% G418 disulfate salt (Nacalai Tesque Inc.)). A strain that grew in static culture for 3 days at 30°C was selected, and transformation efficiency was calculated (the number of colonies per µg of vector; cfu/µg) (Table 6, Condition 15).

The 24 transformed yeast strains obtained under Condition 15 were inoculated on a selective SD G418 agar plate and subjected to static culture at 30°C for 1 day. In order to confirm that vectors had been assembled, chromosome DNA was obtained from the grown strains using Kaneka Easy DNA Extraction Kit Version 2 (Kaneka Corporation) and subjected to PCR with the use thereof as a template, primer 51 (SEQ ID NO: 75) as a forward primer upstream of the CCA38473 terminator, and primer 52 (SEQ ID NO: 76) as a reverse primer downstream of the CCA38473 terminator (Fig. 1). The PCR product was electrophoresed, and assembly efficiency was calculated based on the number of samples in which a band of the size of interest (about 7 kbp) had developed (Table 6, Condition 15).

### <Example 5: Generation of transformed yeast, calculation of transformation efficiency, and calculation of assembly efficiency>

In order to integrate the assembled vectors into the genome, Fragment G1, Fragment G2, and Fragment G3 constructed in Production Example 7 were used to transform the histidine auxotrophic transformed yeast strain Δdnl4Δhis4 constructed in Example 1 in which the gene comprising the nucleotide sequence as shown in SEQ ID NO: 25 (DNL4 gene) had been inactivated in the same manner as in Example 3.

The competent cell suspension obtained in Example 3 (60 µl), a solution of Fragment G1 constructed in Production Example 7 (0.3 pmol), a solution of Fragment G2 constructed in Production Example 7 (0.3 pmol), and a solution of Fragment G3 constructed in Production Example 7 (0.015 pmol) were mixed with each other, and the mixture was transferred into an electroporation cuvette (disposable cuvette electrode, electrode gap of 2 mm, BM Equipment Co., Ltd.). After the mixture was subjected to electroporation at 7.5 kV/cm, 25 µF, and 200 Ω, the resulting cells were suspended in 1 ml of YPD medium and allowed to stand for 1 hour at 30°C. After the resultant was allowed to stand for 1 hour, the yeast culture was harvested (3000× g, 5 minutes, 20°C), and the supernatant was discarded. The yeast culture was resuspended in 1 ml of YNB solution (0.17% Yeast Nitrogen Base without Amino Acids and Ammonium Sulfate (Becton, Dickinson and Company) and 0.1% sodium glutamate (Nacalai Tesque Inc.)), the yeast culture was harvested (3000× g, 5 minutes, 20°C), and 950 µl of the supernatant was discarded. The yeast culture was resuspended in a remaining solution and spread on a selective SD G418His agar plate (0.17% Yeast Nitrogen Base without Amino Acids and Ammonium Sulfate (Becton, Dickinson and Company), 0.1% sodium glutamate (Nacalai Tesque Inc.), 2% glucose, 1.5% agarose, 0.05% G418 disulfate salt (Nacalai Tesque Inc.), and 0.004% L-histidine (Wako Pure Chemical Corporation)). A strain that grew in static culture for 3 days at 30°C was selected, and transformation efficiency was calculated (the number of colonies per µg of vector; cfu/µg) (Table 6, Condition 16).

The 24 transformed yeast strains obtained under Condition 16 were inoculated on a selective SD G418His agar plate and subjected to static culture at 30°C for 1 day. In order to confirm that vectors had been assembled, chromosome DNA was obtained from the grown strains using Kaneka Easy DNA Extraction Kit Version 2 (Kaneka Corporation) and subjected to PCR with the use thereof as a template, primer 51 (SEQ ID NO: 75) as a forward primer upstream of the CCA38473 terminator, and primer 52 (SEQ ID NO: 76) as a reverse primer downstream of the CCA38473 terminator (Fig. 1). The PCR product was electrophoresed, and assembly efficiency was calculated based on the number of samples in which a band of the size of interest (about 7 kbp) had developed (Table 6, Condition 16).

**[Table 6]**

| Condition | Host | Vector (1) | Vector (2) | Molar ratio (Vector (1):Vector (2)) | Transformation efficiency (cfu/µg) | Assembly efficiency (%) |
|---|---|---|---|---|---|---|
| 15 | Wild-type | Fragment G1, Fragment G2 | Fragment G3 | 20 : 1 | 6.7x10³ | 62.5 |
| 16 | Adnl4Δhis4 | Fragment G1, Fragment G2 | Fragment G3 | 20:1 | 5.9x10³ | 100 |

As a result, the assembly efficiency of Condition 15 attained with the use of Fragment G1, Fragment G2, and Fragment G3 was found to be 62.5%. This indicates that vectors are not efficiently assembled in yeast due to, for example, self-circularization of a vector that had occurred because of non-homologous end joining activity of the wild-type strain. Also, the vector may have been integrated into an unintended site of the genome.

In contrast, the assembly efficiency of Condition 16 attained with the use of Fragment G1, Fragment G2, and Fragment G3 was as high as 100%. This indicates that vectors are assembled with each other efficiently in yeast and the assembled vectors are integrated into an intended site of the genome because the Adnl4Δhis4 strain has lost non-homologous end joining activity upon inactivation of the gene comprising the nucleotide sequence as shown in SEQ ID NO: 25 (DNL4 gene) and self-circularization of a vector has been completely suppressed.

The results demonstrated above indicate that the present inventors succeeded in efficiently assembling vectors in yeast by inactivating the gene encoding the DNL4 protein associated with non-homologous end joining by a transformation technique aimed at genome integration.

All publications, patents, and patent applications cited herein are incorporated herein by reference in their entirety.

## Claims

1. A method for assembling two or more types of vectors by a transformation method comprising a step of introducing the two or more types of vectors into a methanol-utilizing yeast strain in which the DNL4 gene has been inactivated.

2. The method according to claim 1, wherein the DNL4 gene is any of the genes (a) to (d) below:
(a) a gene comprising the nucleotide sequence as shown in SEQ ID NO: 25;
(b) a gene comprising a nucleotide sequence of a nucleic acid hybridizing under stringent conditions to a nucleic acid comprising a nucleotide sequence complementary to the nucleotide sequence as shown in SEQ ID NO: 25;
(c) gene comprising a nucleotide sequence having 85% or higher sequence identity to the nucleotide sequence as shown in SEQ ID NO: 25; and
(d) a gene encoding an amino acid sequence having 85% or higher sequence identity to the amino acid sequence as shown in SEQ ID NO: 24.

3. The method according to claim 1 or 2, wherein the methanol-utilizing yeast strain is a yeast strain belonging to the genus *Komagataella* or a yeast strain belonging to the genus *Ogataea.*

4. The method according to any one of claims 1 to 3, wherein the two or more types of vectors each comprise a nucleotide sequence having 85% or higher sequence identity to one another.

5. The method according to any one of claims 1 to 4, wherein at least one of the two or more types of vectors comprises an autonomously replicating sequence (ARS).

6. The method according to any one of claims 1 to 5, wherein at least one of the two or more types of vectors is an autonomously replicating vector.

7. The method according to claim 6, wherein the autonomously replicating vector further comprises an autonomously replicating sequence (ARS) and/or a centromeric DNA sequence derived from a yeast strain belonging to the genus *Komagataella* or a yeast strain belonging to the genus *Ogataea.*

8. A method for producing assembled vectors comprising the method according to any one of claims 1 to 7.

9. Assembled vectors obtained by the method according to any one of claims 1 to 8.

10. A method for producing a transformant comprising a step of transformation with the assembled vectors obtained by the method according to any one of claims 1 to 8.

11. A transformant transformed with the assembled vectors according to claim 9.


SEQUENCE LISTING

<110>  KANEKA CORPORATION

<120>  THE METHOD FOR HIGH EFFICIENT VECTOR ASSEMBLING IN METHYLOTROPHIC
       YEAST

<130>  B170462

<150>  JP 2017-122788
<151>  2017-06-23

<150>  JP 2017-246316
<151>  2017-12-22

<160>  76

<170>  PatentIn version 3.5

<210>  1
<211>  1000
<212>  DNA
<213>  Komagataella pastoris

<400>  1
tctccaccgt cttgatactt tctgaggtgc acagaccaga ggattgtgcg aaccctaaat      60

agttgtatga actcaaattc aagcgctctg tgacggtgcc ggaataatta taggtattgt     120

tgtaatcgtc actggttctg tcgtaacatt tgatgtacct accgggaact ccacatatgg     180

gccttgcaaa acagtcatct aatcttgttt tcaaacgacg cgtgtagaaa ctttcaaaat     240

tggcatacca tggagcaata ccatctttca ccatcacatc tctgaatagt tcagggtgaa     300

acaacttccc aaagaagtct ctaatatggc ctattatgat caaaataagg taattaaggt     360

atgttgcaat caggatgaaa tatggaggtt cgtcctcgat gggaactgga agcggctcgc     420

cagggttatg cttagagaca aagagccatt ctttactggt caaggaccca aattctttct     480

cggccttctc ttgtggtgaa tcattgtcta tgagagcatc tgggatagtt tttgacatga     540

tctttttcag cacggctata tagcaaaaag caaaaaaaaa gaccgaatgg aattatatgg     600

tctaaaaaaa caaactggtg gtaaaataaa aaaaaacgac tggtgggcgg tttcaaagga     660

gactaatgat cttctatgcc cgcggaaata aatagtactc caacgactga actcagcggt     720

attaaagttt gtgaataaaa ttacaaggct tagaaagctt ggttggtctt tcggtatctg     780

tagatggtag agttttgaga acatttcatt tccacagtaa ccaacgaaca cgacccgtga     840

cttccggggg ttggcagatg ttaacgcgcg cgtggtagaa gtttatcttg ggaggtgcta     900

gagggtgctc ttggccttgt tcgctggggg gaagtgtttg tagttaacgt acaactcctc     960

atgactgggg atcagaattt caacttgatt tgccgctaat                          1000

<210>  2
<211>  1007
<212>  DNA

<213>  Komagataella pastoris

<400>  2
cggttaataa aaataatgat ttacatttaa gaagtaacag cacatatata ctgtaagatt      60

aactttgcgt accctaaatt ttactaataa acttaacggg ttgccatagc cttggtaacc     120

acacgtttca atgccaattc agcttttctg aagtcatcac cggaagcatc ttctagctcc     180

aaagcagcca aagcgtcaga caaagcagac tcgatcttgt ccttagcact tctctttagc     240

ttggaagaca aaattgggtc agtgatggta gactcaatgg aggaaacgta agcctccaac     300

ttctgtttgg attcgtgacg gttagcgaag tcctcgtcag ccttcttgaa cttgtcagca     360

tcgttgatca tcttttcgat ctcggaagaa gacaatctac caatagagtt agaaatagtg     420

atgttggcag atcttccggt agacttctcg acagcggtaa ccttcaagat accgttggca     480

tcaatctcaa agatagcctc caacactggc tcaccagcag acataggagg aatgttcttc     540

aagtcgaact cacccaacaa ggtgttctca gaacagttga cacgctcacc ctggtaaact     600

gggaattgaa cagtggtttg gtggtcgtca acagtggtga aagttcttct cttgatagtt     660

gggacagtgg tgtttcttgg aacaactggg gcaaagacgt taccttgcat ggcaacaccc     720

aaagaaagag ggataacatc caacaacaac aagtccttgg tctcttcaga ggtagattga     780

ccggtcaaaa tagcaccttg aacggcggca ccgtaagcga cagcctcatc agggttgatg     840

gatttctcca attgcttacc atcgaagaag tcagacaaca gcttttggac ctttggaatt     900

ctggtggaac caccaaccaa gacgacgtca tcgaccttgg atttctcgat ctttgagtcc     960

ttcaaaactt gttcaacagg ctccaaagta gacttgaaca aatcagc                  1007


<210>  3
<211>  915
<212>  DNA
<213>  Komagataella pastoris

<400>  3
atgtccattg tgaacactga tctggacgga atcctacctt taatcgccaa aggaaaggtt      60

agagacattt atgcagtcga tgagaacaac ttgctgttcg tcgcaactga ccgtatctcc     120

gcttacgatg tgattatgac aaacggtatt cctgataagg gaaagatttt gactcagctc     180

tcagttttct ggtttgattt tttggcaccc tacataaaga atcatttggt tgcttctaat     240

gacaaggaag tctttgcttt actaccatca aaactgtctg aagaaaaata caaatctcaa     300

ttagagggac gatccttgat agtaaaaaag cacagactga tacctttgga agccattgtc     360

agaggttaca tcactggaag tgcatggaaa gagtacaaga actcaaaaac tgtccatgga     420

gtcaaggttg aaaacgagaa ccttcaagag agcgacgcct ttccaactcc gattttcaca     480

ccttcaacga aagctgaaca gggtgaacac gatgaaaaca tctctattga acaagctgct     540

gagattgtag gtaaagacat ttgtgagaag gtcgctgtca aggcggtcga gttgtattct     600

gctgcaaaaa acctcgccct tttgaagggg atcattattg ctgatacgaa attcgaattt 660

ggactggacg aaaacaatga attggtacta gtagatgaag ttttaactcc agattcttct 720

agattttgga atcaaaagac ttaccaagtg ggtaaatcgc aagagagtta cgataagcag 780

tttctcagag attggttgac ggccaacgga ttgaatggca aagagggcgt agccatggat 840

gcagaaattg ctatcaagag taaagaaaag tatattgaag cttatgaagc aattactggc 900

aagaaatggg cttga 915

<210> 4
<211> 487
<212> DNA
<213> Komagataella pastoris

<400> 4
ttttttgtag aaatgtcttg gtgtcctcgt ccaatcaggt agccatctct gaaatatctg 60

gctccgttgc aactccgaac gacctgctgg caacgtaaaa ttctccgggg taaaacttaa 120

atgtggagta atggaaccag aaacgtctct tcccttctct ctccttccac cgcccgttac 180

cgtccctagg aaattttact ctgctggaga gcttcttcta cggccccctt gcagcaatgc 240

tcttcccagc attacgttgc gggtaaaacg gaggtcgtgt acccgaccta gcagcccagg 300

gatggaaaag tcccggccgt cgctggcaat aatagcgggc ggacgcatgt catgagatta 360

ttggaaacca ccagaatcga atataaaagg cgaacacctt tcccaatttt ggtttctcct 420

gacccaaaga ctttaaattt aatttatttg tccctatttc aatcaattga acaactatca 480

aaacaca 487

<210> 5
<211> 500
<212> DNA
<213> Komagataella pastoris

<400> 5
tcatcggggg tataatacat gtattttgat agaactttga tattattaat tatctgattg 60

aattgtaggt ccctatgctt ttcagaaatt gtgccaccat ttttcattag ttgagcacta 120

taaaacagtt ttcaagattg tagtcgtcta actcctcgct tggttgggcg gtaggagttc 180

agtcggaaga taaagatctc ttcgcgataa ggtttagact acattcagtt tctccttgat 240

ctattacata ctcccaagta tgactaacga tgctccgtta tgggttctag gctatggttc 300

tctaatattt aagccgcctc ctcatgcgac acataagatt ccaggcaaaa tctcaggttt 360

tgtcagacgg ttttggcaga gttctacaga tcatagaggt acaccggaga gcccaggaag 420

agtggtaacc ttaattccgt acgcagatat agtaagggat caccggtttc tagaaagtgt 480

tcattacttt gagtgtaacg 500

<210>  6
<211>  6655
<212>  DNA
<213>  Komagataella pastoris

<400>  6
ccaatcaaac aaggtgactt gcgcgaagca atgatttgtg gatgggctgc ggtatggcag      60

cataacaatg caacgctatt tcagaaattg taaagtgtaa aggaaatatt caaccctata     120

aagaatctac cgaaacgtag gataaataat ttccacagag tacacttttg gtttttatgg     180

acggcgttga gttgcacaga tgatggaata ttgtgataaa atacgctata taattttgga     240

gaccctaaga tcaagtggac taggtcgatt gcgtatatta gggtattaaa gacttactta     300

attctataag agctcggcaa gctatattct cagttccttc gcagtcgacc ttcagtgccc     360

aaaactttaa agtggacggg tgttttctaa agattgctat tgttcaattg cccggttttt     420

taaccgctga aaaccagtgg caagagtgga ctacgtgcgc attgcaattt gtataatcca     480

tataattttg ttccacggcc ttaagttatt gtactgtttg ctcaattttg aagcggaata     540

tccgtcctag tctcgaatcc ttatttaagt accggtatca tttgcatatc ctctcagccc     600

tcaaacgccc attctttata gtaatggtta tgtattaatc gactacgatg attgactcag     660

ggaggcacga atagtataat ttgtaacttg cagtgggaag ctaatttcgg gtgtcttgaa     720

agacgaggtt aaaaatttga acccaaaccg attttaccgg agatcctacc tcaaattaga     780

acaactttaa agctttcaag gaattgaaat tccatcttta tagaccggta attctttgaa     840

gagtaatcaa tgtaagagca agttctaaag agaggatcca aattccttct gatagcaatt     900

gtaaggagct gcaaagttca acataatatt tgcgatagta accgtcctac ggatacttaa     960

atcggtatca aagagtgacc aatcctgaat cagtgtagtg caaaaagaaa taacgatgat    1020

aaacgaatca agattctgat gagttgtcca agacggggct atcataatat ttaaaaaata    1080

gaacctacta atttaacaag gtaaatctca tagatttaag tttcacgact agcttagcta    1140

ccactcaaat aagctcccgc ttgttcattc ggctggggtc tccaatctcc atctccaatg    1200

gatatttttt ttttaagtgg agagagatgt tcagtaaact gtgagttcag ttgtcagaag    1260

caaccaatat ttaccatgga gacgaataag cagatattat atactctatt tttacagccc    1320

ttttgtataa tcctcaggga tggttggcgc ttccctgtgt tccaagtaac cacactcttc    1380

aagttggaaa tgcaaaaggt cccaatgcat gctcccaagt catactgaat acagagatag    1440

attctttgaa ttggatcgat ctctaatgaa attcatggat gagaagagag tgttaaaaac    1500

tttgccccaa ctaaatactt tatctgaatg aatcattaaa gaacacgaca agttcagtat    1560

gttgcttgga gcctccgatt cacagtcttg taagtagtgc gtgttttcaa atatttgcat    1620

tagcaatccc tccaagtagc ccacataggg accaaaaaaa tagtatagtt tgttttgggg    1680

ggaagggagt aggaattgct ttcgaaatgg ttaactcctg cttagtttca tttaaaaaat 1740

gaatagagaa gtagatacag aatactttgt aagactgctt acatgaatga tgccagagag 1800

tgtgccgaaa gatctacatg ctactaaatt tactatcaat agggataaaa tgtattaggc 1860

tacctactat gccgtattgg aaaggcgaag tgctttgtat gcaagggatt ggggcatagg 1920

caaacggatc tatgacaaga gatacgaaat tacttctgtt acccactctg ctcctaccaa 1980

accaaatata tctgtgacca aaaatatcat ttgtcttgtg ctccacatag cggccaggct 2040

caaagagaac actagatctg ctttcagaca ttaccgcgtg caaatatgac aagtcactag 2100

gtgatgaact agtcgataaa accaaaaacg atctgatcaa taacttgaca acatcgttct 2160

tatcggattt ctagctcttg aatagtcaaa tgctgagttc aattttaaag tgaatatttt 2220

tcaccgaagt cgattttagc ccattagtca aacaacagaa gagaaaagac aaaccttctt 2280

tttcatgaca catgaaacaa atcacaaaaa ttgagtcata ttacggtttc aatttctgct 2340

gtttgtataa gtcattggct ggcaaacaca tctcctggcc tcatactgaa aatatatgaa 2400

gataactgct tctaccaatt tcaattgatc aaggttctac ccatacgtat agttccagag 2460

catgatgtta gggaaactta caggatgatg gtgggccaca gcataatatc aacaatacct 2520

aacaatcggt atttaattca tagatattca ataccaattc tccacgatga gaaggggcga 2580

ctcctcttct tggacgttct gaaaatacag gtacgctcac gtcagtgcct gagcacatac 2640

agcgacgtta gtatcaatct tcctgggaca tctctaatgc tctaaaatga agaaaaagta 2700

aaattagtat cagttggcca tgcgaccaag cctgttggaa cgcaaaagca tatccgaatt 2760

atggttggaa attaccttcg accttcaaac actaaactct tcacagggct attattccag 2820

ctattgcaga acttcatacg aatttgggct tgctaaaaat tggcaggatc gttcttcaat 2880

aactgaatac agtattgtac ctattcaagc agttagtcca tacttagggc cttcctagcc 2940

tgtccagtct accattaacg aaagttctga gctgtgtcta tccaaattaa tttgtgtcag 3000

aacctacgta accactataa gtctcataac gtttttgaaa tagataaaat tagtttgttt 3060

gatcattcaa gcacattata gataatattc caaaaagaaa gtttcaaggt gccccttgga 3120

catataaaac tttgccacgg tgctaaactt aaaagaatga tcttccaaaa atacggaata 3180

gaataatcta tttttagaag agaagactaa aaatcgagcg atactatgaa tgtctgttac 3240

aaactgaatt tttgcacgta acgaatactt tgaaacaatc ctcaaaatct tgttttggtg 3300

tttgtcgagt agtctttacc aggccgaatc ctgtagttaa aaataatttt ctatttggac 3360

aactaagggt atcccctcaa gtttggcttt tgtgtacctt gttcggaaag gctccgttct 3420

gcaattctgc agcaaccttg tcattagctg tgtaatgctc gctggtgagt agcataagtc 3480

attccaatgg taactaccaa gcttgtaata taaccaccag cacctccatt cagtagtagc 3540

aatgagcaag gtcggagatt gcttttaagc gaggtttcaa agtaatcttg tagttttgct 3600

ttgtcgaaaa atggccacgg cttacacata cagttcgtcc accagatctt taggcttcat 3660

caacaaagtt ttcgacttcg ttgtctatag gttttattct ttatcagcgt cagactatga 3720

tcgtactagt ttgagctgaa gcccatcagg atgtttacca ctccagtttt gtaatttccg 3780

cggctatcta gggctacgtt gtccattgcc gagtttatat atgacatcac attaggcagt 3840

aattctccat gcttcttttt cgataatgtt ttggtctagg ttcgtgcatg aaattttttc 3900

ttcatccaga ctatctctgc gctgcactgt tcacatagcc aactgatact gatttcactt 3960

tttcttcatt ttagagcatt agagatgtcc caggaagatt gatactaacg tcgctgtatg 4020

tgctcaggca ctgacgtgag cgtacctgta ttttcagaac gtccaagaag aggagtcgcc 4080

ccttctcatc gtggagaatt ggtattgaat atctatgaat taaataccga ttgttaggta 4140

ttgttgatat tatgctgtgg cccacatcat ccctgtaagt ttccctaaca tcatgctctg 4200

gaactatacg tatgggtaga accttgatca attgaaattg gtagaagcag ttatcttcat 4260

atattttcag tatgaggcca ggagatgtgt ttgccagcca atgacttata caaacagcag 4320

aaattgaaac cgtaatatga ctcaattttt gtgatttgtt tcatgtgtca tgaaaaagaa 4380

ggtttgtctt ttctcttctg ttgtttgact aatgggctaa aatcgacttc ggtgaaaaat 4440

attcacttta aaattgaact cagcatttga ctattcaaga gctagaaatc cgataagaac 4500

gatgttgtca agttattgat cagatcgttt ttggttttat cgactagttc atcacctagt 4560

gacttgtcat atttgcacgc ggtaatgtct gaaagcagat ctagtgttct ctttgagcct 4620

ggccgctatg tggagcacaa gacaaatgat atttttggtc acagatatat ttggtttggt 4680

aggagcagag tgggtaacag aagtaatttc gtatctcttg tcatagatcc gtttgcctat 4740

gccccaatcc cttgcataca aagcacttcg cctttccaat acggcatagt aggtagccta 4800

atacatttta tccctattga tagtaaattt agtagcatgt agatctttcg gcacactctc 4860

tggcatcatt catgtaagca gtcttacaaa gtattctgta tctacttctc tattcatttt 4920

ttaaatgaaa ctaagcagga gttaaccatt tcgaaagcaa ttcctactcc cttcccccca 4980

aaacaaacta tactattttt ttggtcccta tgtgggctac ttggagggat tgctaatgca 5040

aatatttgaa aacacgcact acttacaaga ctgtgaatcg gaggctccaa gcaacatact 5100

gaacttgtcg tgttctttaa tgattcattc agataaagta tttagttggg gcaaagtttt 5160

taacactctc ttctcatcca tgaatttcat tagagatcga tccaattcaa agaatctatc 5220

tctgtattca gtatgacttg ggagcatgca ttgggacctt ttgcatttcc aacttgaaga 5280

gtgtggttac ttggaacaca gggaagcgcc aaccatccct gaggattata caaaagggct 5340

gtaaaaatag agtatataat atctgcttat tcgtctccat ggtaaatatt ggttgcttct 5400

gacaactgaa ctcacagttt actgaacatc tctctccact taaaaaaaaa atatccattg 5460

gagatggaga ttggagaccc cagccgaatg aacaagcggg agcttatttg agtggtagct 5520

aagctagtcg tgaaacttaa atctatgaga tttaccttgt taaattagta ggttctattt 5580

tttaaatatt atgatagccc cgtcttggac aactcatcag aatcttgatt cgtttatcat 5640

cgttatttct ttttgcacta cactgattca ggattggtca ctctttgata ccgatttaag 5700

tatccgtagg acggttacta tcgcaaatat tatgttgaac tttgcagctc cttacaattg 5760

ctatcagaag gaatttggat cctctcttta gaacttgctc ttacattgat tactcttcaa 5820

agaattaccg gtctataaag atggaatttc aattccttga aagctttaaa gttgttctaa 5880

tttgaggtag gatctccggt aaaatcggtt tgggttcaaa tttttaacct cgtctttcaa 5940

gacacccgaa attagcttcc cactgcaagt tacaaattat actattcgtg cctccctgag 6000

tcaatcatcg tagtcgatta atacataacc attactataa agaatgggcg tttgagggct 6060

gagaggatat gcaaatgata ccggtactta aataaggatt cgagactagg acggatattc 6120

cgcttcaaaa ttgagcaaac agtacaataa cttaaggccg tggaacaaaa ttatatggat 6180

tatacaaatt gcaatgcgca cgtagtccac tcttgccact ggttttcagc ggttaaaaaa 6240

ccgggcaatt gaacaatagc aatctttaga aaacacccgt ccactttaaa gttttgggca 6300

ctgaaggtcg actgcgaagg aactgagaat atagcttgcc gagctcttat agaattaagt 6360

aagtctttaa caccctaata tacgcaatcg acctagtcca cttgatctta gggtctccaa 6420

aattatatag cgtattttat cacaatattc catcatctgt gcaactcaac gccgtccata 6480

aaaaccaaaa gtgtactctg tggaaattat ttatcctacg tttcggtaga ttctttatag 6540

ggttgaatat ttcctttaca ctttacaatt tctgaaatag cgttgcattg ttatgctgcc 6600

ataccgcagc ccatccacaa atcattgctt cgcgcaagtc accttgtttg attgg 6655

<210> 7
<211> 28
<212> DNA
<213> Artificial Sequence

<220>
<223> Primer 1

<400> 7
actgctgcag tctccaccgt cttgatac 28

<210> 8
<211> 30
<212> DNA
<213> Artificial Sequence

<220>
<223> Primer 2

<400> 8
tcgcggatcc attagcggca aatcaagttg 30

<210>  9
<211>  34
<212>  DNA
<213>  Artificial Sequence

<220>
<223>  Primer 3

<400>  9
cgccggatcc cggttaataa aaataatgat ttac                                    34


<210>  10
<211>  33
<212>  DNA
<213>  Artificial Sequence

<220>
<223>  Primer 4

<400>  10
tgacgtcggt accgctgatt tgttcaagtc tac                                     33


<210>  11
<211>  28
<212>  DNA
<213>  Artificial Sequence

<220>
<223>  Primer 5

<400>  11
atctggtacc gggtgctatc gttttgtg                                           28


<210>  12
<211>  29
<212>  DNA
<213>  Artificial Sequence

<220>
<223>  Primer 6

<400>  12
acatggtacc ccatactgag ccaaaagac                                          29


<210>  13
<211>  32
<212>  DNA
<213>  Artificial Sequence

<220>
<223>  Primer 7

<400>  13
ttaggcgcgc cttttttgta gaaatgtctt gg                                       32


<210>  14

<211>  47
<212>  DNA
<213>  Artificial Sequence

<220>
<223>  Primer 8

<400>  14
ttaactagtt gtgttttgat agttgttcaa ttgattgaaa tagggac                    47


<210>  15
<211>  30
<212>  DNA
<213>  Artificial Sequence

<220>
<223>  Primer 9

<400>  15
ttactcgagt catcgggggt ataatacatg                                       30


<210>  16
<211>  35
<212>  DNA
<213>  Artificial Sequence

<220>
<223>  Primer 10

<400>  16
ttattaatta acgttacact caaagtaatg aacac                                 35


<210>  17
<211>  40
<212>  DNA
<213>  Artificial Sequence

<220>
<223>  Primer 11

<400>  17
ttagcggccg cccaatcaaa caaggtgact tgcgcgaagc                            40


<210>  18
<211>  30
<212>  DNA
<213>  Artificial Sequence

<220>
<223>  Primer 12

<400>  18
gaaatcagta tcagttggct atgtgaacag                                       30


<210>  19
<211>  28
<212>  DNA
<213>  Artificial Sequence

<220>
<223>  Primer 13

<400>  19
aaaattagta tcagttggcc atgcgacc                                          28


<210>  20
<211>  38
<212>  DNA
<213>  Artificial Sequence

<220>
<223>  Primer 14

<400>  20
ttatctagac caatcaaaca aggtgacttg cgcgaagc                               38


<210>  21
<211>  855
<212>  DNA
<213>  Artificial Sequence

<220>
<223>  Zeo

<400>  21
cccacacacc atagcttcaa aatgtttcta ctcctttttt actcttccag attttctcgg       60

actccgcgca tcgccgtacc acttcaaaac acccaagcac agcatactaa attttccctc      120

tttcttcctc tagggtgtcg ttaattaccc gtactaaagg tttggaaaag aaaaaagaga      180

ccgcctcgtt tctttttctt cgtcgaaaaa ggcaataaaa atttttatca cgtttctttt      240

tcttgaaatt ttttttttta gtttttttct ctttcagtga cctccattga tatttaagtt       300

aataaacggt cttcaatttc tcaagtttca gtttcatttt tcttgttcta ttacaacttt      360

ttttacttct tgttcattag aaagaaagca tagcaatcta atctaagggg cggtgttgac      420

aattaatcat cggcatagta tatcggcata gtataatacg acaaggtgag gaactaaacc      480

atggccaagt tgaccagtgc cgttccggtg ctcaccgcgc gcgacgtcgc cggagcggtc      540

gagttctgga ccgaccggct cgggttctcc cgggacttcg tggaggacga cttcgccggt      600

gtggtccggg acgacgtgac cctgttcatc agcgcggtcc aggaccaggt ggtgccggac      660

aacaccctgg cctgggtgtg ggtgcgcggc ctggacgagc tgtacgccga gtggtcggag      720

gtcgtgtcca cgaacttccg ggacgcctcc gggccggcca tgaccgagat cggcgagcag      780

ccgtgggggc gggagttcgc cctgcgcgac ccggccggca actgcgtgca cttcgtggcc      840

gaggagcagg actga                                                       855


<210>  22
<211>  795
<212>  DNA

<213>  Artificial Sequence

<220>
<223>  G418

<400>  22
atgattgaac aagatggatt gcacgcaggt tctccggccg cttgggtgga gaggctattc      60

ggctatgact gggcacaaca gacaatcggc tgctctgatg ccgccgtgtt ccggctgtca     120

gcgcaggggc gcccggttct ttttgtcaag accgacctgt ccggtgccct gaatgaactg     180

caggacgagg cagcgcggct atcgtggctg gccacgacgg gcgttccttg cgcagctgtg     240

ctcgacgttg tcactgaagc gggaagggac tggctgctat tgggcgaagt gccggggcag     300

gatctcctgt catctcacct tgctcctgcc gagaaagtat ccatcatggc tgatgcaatg     360

cggcggctgc atacgcttga tccggctacc tgcccattcg accaccaagc gaaacatcgc     420

atcgagcgag cacgtactcg gatggaagcc ggtcttgtcg atcaggatga tctggacgaa     480

gagcatcagg ggctcgcgcc agccgaactg ttcgccaggc tcaaggcgcg catgcccgac     540

ggcgaggatc tcgtcgtgac ccatggcgat gcctgcttgc cgaatatcat ggtggaaaat     600

ggccgctttt ctggattcat cgactgtggc cggctgggtg tggcggaccg ctatcaggac     660

atagcgttgg ctacccgtga tattgctgaa gagcttggcg gcgaatgggc tgaccgcttc     720

ctcgtgcttt acggtatcgc cgctcccgat tcgcagcgca tcgccttcta tcgccttctt     780

gacgagttct tctga                                                      795


<210>  23
<211>  720
<212>  DNA
<213>  Artificial Sequence

<220>
<223>  GFP

<400>  23
atggtttcta agggtgaaga gttgttcact ggtgttgttc caatcttggt tgagttggac      60

ggtgacgtta acggacacaa gttctctgtt tctggtgaag gtgagggtga cgctacttac     120

ggaaagttga ctttgaagtt catctgtact actggtaagt tgccagttcc atggccaact     180

ttggttacta ctttgactta cggtgttcag tgtttctcca gatacccaga ccacatgaag     240

cagcacgatt tcttcaagtc tgctatgcca gagggttacg ttcaagagag aactatcttc     300

ttcaaggacg acggtaacta caagactaga gctgaggtta agttcgaggg tgacacattg     360

gttaacagaa tcgagttgaa gggtatcgac ttcaaagagg acggaaacat cttgggtcac     420

aagttggagt acaactacaa ctcccacaac gtttacatca tggctgacaa gcagaagaac     480

ggtatcaagg ttaacttcaa gatcagacac aacatcgagg acggttccgt tcaattggct     540

gaccactacc aacagaacac tccaattggt gacggtccag ttttgttgcc agacaaccac     600

tacttgtcca ctcaatccgc tttgtccaag gacccaaacg agaagagaga tcacatggtt 660

ttgttggagt tcgttactgc tgctggtatc actttgggta tggacgagtt gtacaagtaa 720

<210> 24
<211> 932
<212> PRT
<213> Komagataella pastoris

<400> 24

Met Ser Glu Glu Leu Lys Glu Pro Lys Asn Arg Ala Pro Leu Pro Asn
1               5                   10                  15

Phe Ser His Leu Val Tyr Asp Leu Phe Glu Lys Leu Asp Thr Cys Val
            20                  25                  30

Lys Ser Pro His Ile Pro Glu Ser Leu Arg Ser Arg Lys Thr Lys Ile
        35                  40                  45

Ile Glu Gln Phe Val Gln Lys Trp Arg Phe Glu Ile Gly Asp Asn Ile
    50                  55                  60

Tyr Pro Ala Ser Arg Leu Ile Phe Pro Asn Leu Asp Lys Arg His Tyr
65                  70                  75                  80

Asn Ile Lys Asp Phe Thr Leu Ile Lys Phe Ile Leu Asp Ile Phe Gln
                85                  90                  95

Val Pro Lys Asp Ser Glu Asp Thr Lys Ile Leu Arg Asn Trp Lys Gly
            100                 105                 110

Lys Tyr Glu Asn Arg Met Arg Ser Asn Lys Asn Asp Leu Ala Gln Leu
        115                 120                 125

Leu Thr Gly Val Ile Lys Ala Arg Arg Gly Ala Val Leu Asp Arg Pro
    130                 135                 140

Glu Ile Ser Ile Asp Arg Leu Asn Glu Leu Leu Asp Thr Met Thr Arg
145                 150                 155                 160

Pro Glu Thr Lys Pro Ser Asp Gln Lys Lys Ile Leu Thr Glu Ile Phe
                165                 170                 175

Asn His Val Asp Asn Ile Glu Leu Lys Trp Phe Leu Arg Ile Leu Leu
            180                 185                 190

Lys Arg Lys Leu Met Gly Gly Met Asp Phe Thr Phe Phe Arg Gln Trp
        195                 200                 205

His Pro Asp Ala Val Glu Leu Leu Gln Leu Val Asn Asp Leu Lys Thr
    210                 215                 220

Val Phe Trp Glu Leu Thr Asp Arg Leu Ile Pro Leu Pro Glu Ala Gly
225                 230                 235                 240

Arg Val Ile Arg Val Gly Arg Ala Phe Arg Pro Gln Leu Ala Met Arg
                245                 250                 255

Pro Asn Arg Thr Tyr Asp Gln Ile Ala Thr Leu Phe Asp Asn Asn Phe
            260                 265                 270

Tyr Ile Glu Asp Lys Leu Asp Gly Glu Arg Ile Leu Met His Ile Ser
        275                 280                 285

Leu Asn Asp Thr Thr Gln Gln His Glu Phe Lys Tyr Phe Ser Arg Ser
    290                 295                 300

Gly Thr Asp Tyr Thr Tyr Leu Tyr Gly Ser Asn Tyr Glu Glu Gly Ala
305                 310                 315                 320

Phe Ser Pro Phe Ile Lys Gly Cys Phe Ile Asp Leu Lys Asp Asp Thr
                325                 330                 335

Lys Glu Ile Arg Ser Met Val Leu Asp Gly Glu Phe Leu Val Tyr Asp
            340                 345                 350

Asn Lys Arg Asn Val Ile Leu Pro Phe Gly Ala Leu Lys Ser Ala Ser
        355                 360                 365

Leu Thr Arg Leu Met Glu Glu Lys Asn Asp Ser Glu Asp Ser Gln Glu
    370                 375                 380

Thr Gln Val Tyr Phe Val Val Tyr Asp Leu Val Phe Leu Asn Gly Thr
385                 390                 395                 400

Ser Phe Gln Asp Lys Ala Leu Lys Val Arg Arg Asn Leu Leu Asn Lys
                405                 410                 415

Ile Leu Ala Asn Pro Ile Pro Asn Arg Ile Glu Val Ile Lys Tyr Thr
            420                 425                 430

Glu Gly His Val Gly Ser Asp Ile Glu Ala Ala Met Arg Ser Ala Ile
        435                 440                 445

His Asp Asn Arg Glu Gly Ile Val Val Lys Asn Pro Lys Ser Lys Tyr

450                 455                 460

Tyr Ile Ala Ser Arg Asn Leu Asn Trp Val Lys Ile Lys Pro Glu Tyr
465                 470                 475                 480

Leu Glu Glu Phe Gly Glu Asn Ile Asp Leu Ile Ile Leu Gly Lys Arg
                485                 490                 495

Lys Gly Leu Lys Asn Ala Tyr Ile Cys Gly Leu Arg Glu Ser Leu Gly
            500                 505                 510

Asn Asp Arg Tyr Arg Phe Leu Ser Leu Ala Arg Ile Ala Asn Gly Phe
        515                 520                 525

Thr Gly Gln Met Tyr Glu Glu Ile Glu His Ser Leu Gly Ser Lys Trp
    530                 535                 540

Val Asn Ile Arg Lys Thr Gln Pro Pro Tyr Pro Tyr Ile Asp Leu Gly
545                 550                 555                 560

Glu Ile Ser Ser Met Val Asp Tyr Trp Val Asp Pro Lys Glu Ser Val
                565                 570                 575

Val Leu Glu Ile Lys Ala Arg Ser Ile Asp Ile Gly Asp Gly Gly Lys
            580                 585                 590

Arg Tyr Ala Ala Gly Thr Thr Leu Tyr Asn Ala Tyr Cys Val Arg Ile
        595                 600                 605

Arg Ser Asp Lys Asp Phe Leu Ser Cys Ser Thr Phe Ala Asp Tyr Lys
    610                 615                 620

Glu Ile Lys Thr Ser Lys Ser Arg His Lys Thr Glu Lys Thr His Ser
625                 630                 635                 640

Leu Met Ser Lys Arg Lys Arg Thr Ile Val Thr Glu Pro Ile Leu Ala
                645                 650                 655

Lys Gln Ser Val Ser Ser Lys Ser Ser Ile Phe Lys Asp Tyr Ile Phe
            660                 665                 670

Tyr Val Leu Ser Asp Met Leu Gly Glu Lys Arg Tyr Ser Ile Ala Gln
        675                 680                 685

Leu Glu Ala Leu Leu Val Glu Asn Gly Gly Arg Ile Thr Lys Asn Asp
    690                 695                 700

Cys Val Ala Leu Ala Asp Gly Glu Arg Leu Val Leu Leu Ser Asp Lys
705                 710                 715                 720

Ile Thr Phe Lys Val Thr Glu Tyr Phe Asn Lys Gly Leu Asn Val Phe
                725                 730                 735

Arg Pro Gly Phe Ile Leu Lys Cys Leu Gln Leu Lys Thr Ile Ile Pro
            740                 745                 750

Leu Gln Ile Asn Asp Ile Leu Leu Ala Asp Glu Ser Leu Ile Glu Lys
        755                 760                 765

Val Lys Lys Asn Ser Asp Arg Phe Gly Asp Ser Tyr Thr Ser Pro Ile
    770                 775                 780

Asp Arg Lys Glu Leu Arg Met Leu Met Lys Gly Ile Pro Lys Ile Lys
785                 790                 795                 800

Leu Glu His Pro Leu Lys Phe Pro Leu Leu Tyr Asn Leu Phe Lys Gly
                805                 810                 815

Leu Lys Phe Tyr Ile Ala Arg Pro Cys Tyr Arg Ser Pro Phe Leu Thr
            820                 825                 830

Asn Val Met Ser Pro Tyr Leu Leu Glu Val Ile Val Ile Leu Gly Gly
        835                 840                 845

Phe Leu Thr Thr Asp Ile Phe Gln Cys Ser Phe Ile Ile Val Asp Thr
    850                 855                 860

Ser Thr Thr Phe Asn Tyr Ser Trp Leu Thr Glu Glu Ala Thr Ile Gln
865                 870                 875                 880

Asp Glu Val Gly Ser Leu Arg Lys Lys Leu Gly Asp Asp Leu Pro Ile
                885                 890                 895

Val Lys Gly Val Leu Asp Lys Val Pro Arg Ile Val Glu Lys Glu Trp
            900                 905                 910

Val Phe Lys Cys Ile Gln Glu Gly Thr Ile Val Ala Ala Asp Gly Tyr
        915                 920                 925

Asn Tyr Ile Gly
    930

<210>  25
<211>  2799
<212>  DNA

<213>  Komagataella pastoris

<400>  25
atgagtgaag agttgaagga gccaaaaaac agagcccctt tacctaattt cagccatcta      60

gtttatgacc ttttcgaaaa attggataca tgtgtcaaat cgcctcatat tcctgaatct     120

ctcagaagtc gaaagaccaa gataatagag cagtttgtcc aaaaatggag atttgagata     180

ggtgataaca tatatcctgc ctcaagactt atttttccca atttagataa gcgtcactat     240

aatatcaagg attttacatt gatcaagttc atattggaca tcttccaagt tccaaaagac     300

tccgaagata cgaagatttt acgtaactgg aaagggaagt atgagaatag aatgcgctcc     360

aacaaaaatg atctagctca attactcact ggagttatta aagctaggag aggtgctgtt     420

ttggatcggc cagaaatttc tattgacaga ctgaacgaac ttttggatac aatgactcgc     480

cctgaaacaa aaccgtctga ccaaaaaaag attttgacgg agattttcaa tcatgtagat     540

aatattgaac tgaaatggtt tcttcggatc ctactgaaaa ggaagttaat gggtggtatg     600

gattttactt tttttaggca gtggcatcca gatgctgtag aattacttca actggttaat     660

gacctgaaaa ctgtattctg ggaactgact gatcggttga ttccattgcc tgaagcgggc     720

agagtcatca gagttgggcg tgcattcagg cctcaactgg caatgcgacc caatagaact     780

tacgatcaaa tagctactct atttgacaat aatttctaca ttgaagataa actagatggc     840

gaacggattt tgatgcacat cagcttgaat gacacaaccc aacaacatga attcaagtac     900

ttttcaagga gtggaactga ctatacatat ttatacgggt caaactacga agaaggtgca     960

ttctctcctt tcatcaaagg ttgcttcata gatctcaaag atgacacaaa agaaatccga    1020

tctatggttt tggatgggga gtttctcgtt tatgataaca aaagaaatgt tattctcccc    1080

tttggggctc tcaagagtgc cagtttaacg agattgatgg aggagaaaaa cgattcggaa    1140

gactctcaag agacgcaagt gtatttcgtt gtttatgatc ttgtattctt aaatggtaca    1200

tcatttcaag acaaagctct caaggtaagg cggaatttac tgaataaaat tcttgcaaat    1260

ccaatcccta acagaataga agttatcaaa tacacggagg gtcatgtcgg atcggatatc    1320

gaagctgcca tgagaagtgc tatacacgac aatcgtgaag gaatcgtagt gaagaatcca    1380

aaatcaaagt attatattgc atccagaaat ctcaactggg tcaagataaa acctgagtac    1440

ttggaagaat ttggagaaaa tatagatttg attattctag gcaaaagaaa aggactgaaa    1500

aacgcctata tctgtggact cagagagagt cttggtaatg accggtacag attcttgtca    1560

ctggccagaa ttgccaatgg atttacaggg caaatgtatg aagaaattga acattcactg    1620

gggagtaaat gggtcaatat aaggaagacg caaccacctt atccatacat tgatctcgga    1680

gagatatcat cgatggtgga ctattgggtt gatcccaaag aatctgtcgt tttagagatc    1740

aaggctcgtt caattgatat tggtgatggc gggaaacgtt acgcagctgg taccactctt    1800

tacaatgcgt attgtgtaag aattagatct gataaagact tcttatcatg ttcgactttt 1860

gcagattaca aagaaataaa aacttcaaag agtagacaca aaacagagaa aacacacagt 1920

ctaatgagta aacgaaagag gacaatagtc actgaaccaa ttctcgctaa acaaagtgta 1980

tcttcgaaga gcagcatatt caaggattac atcttttatg ttttaagtga catgttgggt 2040

gaaaaacgat actctattgc tcagctggag gctttgctgg tcgagaatgg tggcagaata 2100

actaagaatg attgcgtagc tcttgcagat ggcgagaggc ttgttttact tagtgacaag 2160

attactttca aagtcacaga gtactttaat aaaggtctaa atgtctttcg accaggcttc 2220

attctgaagt gtcttcagct gaaaactata attcctctac aaattaacga catattactg 2280

gcggatgagt ctttgataga aaaggtaaag aaaaactccg accgatttgg ggatagttat 2340

acctctccca ttgaccgtaa ggagttgcga atgcttatga aaggtatacc taagataaag 2400

ttagaacacc cactaaaatt tccactactg tacaacttgt tcaagggcct gaagttttat 2460

atcgcaagac catgctatag atctccattt ctgacaaatg ttatgagtcc atacttgtta 2520

gaagtaatag tgattctagg aggatttctt acaactgata ttttccaatg ttcatttata 2580

atcgtggata cttccaccac attcaactat tcctggttga ctgaggaggc gaccatacag 2640

gatgaagttg ggtctctaag aaaaaagctg ggtgatgatc taccaatcgt caaaggtgtt 2700

ctagataaag taccccggat agtagagaaa gagtgggtgt tcaaatgtat ccaggaaggg 2760

acaatcgttg ctgcggatgg ctataactat atcggttaa 2799

<210> 26
<211> 94
<212> DNA
<213> Artificial Sequence

<220>
<223> Primer 15

<400> 26
ttaaagcttt ctagagcggc cgcggcgcgc ctgcggcctg tttggcctta attaagcgat 60

cgcggcctgt ttggccccca cacaccatag cttc 94

<210> 27
<211> 31
<212> DNA
<213> Artificial Sequence

<220>
<223> Primer 16

<400> 27
ttagaattct cagtcctgct cctcggccac g 31

<210> 28
<211> 30

<212>  DNA
<213>  Artificial Sequence

<220>
<223>  Primer 17

<400>  28
ttaactagta tggtttctaa gggtgaagag                                        30


<210>  29
<211>  38
<212>  DNA
<213>  Artificial Sequence

<220>
<223>  Primer 18

<400>  29
ttactcgagt tacttgtaca actcgtccat acccaaag                               38


<210>  30
<211>  22
<212>  DNA
<213>  Artificial Sequence

<220>
<223>  Primer 19

<400>  30
atgattgaac aagatggatt gc                                                22


<210>  31
<211>  22
<212>  DNA
<213>  Artificial Sequence

<220>
<223>  Primer 20

<400>  31
tcagaagaac tcgtcaagaa gg                                                22


<210>  32
<211>  52
<212>  DNA
<213>  Artificial Sequence

<220>
<223>  Primer 21

<400>  32
ttcaatcaat tgaacaacta tcaaaacaca atgattgaac aagatggatt gc               52


<210>  33
<211>  52
<212>  DNA
<213>  Artificial Sequence

<220>
<223>  Primer 22

<400>  33
atcaaaatac atgtattata cccccgatga tcagaagaac tcgtcaagaa gg          52


<210>  34
<211>  82
<212>  DNA
<213>  Artificial Sequence

<220>
<223>  Primer 23

<400>  34
agactttaaa tttaatttat ttgtccctat ttcaatcaat tgaacaacta tcaaaacaca      60

atgattgaac aagatggatt gc                                               82


<210>  35
<211>  82
<212>  DNA
<213>  Artificial Sequence

<220>
<223>  Primer 24

<400>  35
caatcagata attaataata tcaaagttct atcaaaatac atgtattata cccccgatga      60

tcagaagaac tcgtcaagaa gg                                               82


<210>  36
<211>  24
<212>  DNA
<213>  Artificial Sequence

<220>
<223>  Primer 25

<400>  36
aggagttaga cgactacaat cttg                                             24


<210>  37
<211>  164
<212>  DNA
<213>  Artificial Sequence

<220>
<223>  PARS1

<400>  37
tcgagataag ctgggggaac attcgcgaaa atgaaacaag tcggctgtta tagtatattt      60

attataatat tgaaagatct caaaagacta cttatttttg aatgaaccaa gtatgaaatc      120

aacctatttg gggttgacca aaataagtaa atattaattg tcga                       164

<210>  38
<211>  37
<212>  DNA
<213>  Artificial Sequence

<220>
<223>  Primer 26

<400>  38
ttagcggccg cttcgagata agctggggga acattcg                                37


<210>  39
<211>  40
<212>  DNA
<213>  Artificial Sequence

<220>
<223>  Primer 27

<400>  39
ttagcggccg ctcgacaatt aatatttact tattttggtc                             40


<210>  40
<211>  30
<212>  DNA
<213>  Artificial Sequence

<220>
<223>  MCS

<400>  40
ggtaccccta gggatatcac gcgttgtaca                                        30


<210>  41
<211>  1000
<212>  DNA
<213>  Komagataella pastoris

<400>  41
catgttggta ttgtgaaata gacgcagatc gggaacactg aaaaataaca gttattattc      60

gagatctaac atccaaagac gaaaggttga atgaaacctt tttgccatcc gacatccaca     120

ggtccattct cacacataag tgccaaacgc aacaggaggg gatacactag cagcagaccg     180

ttgcaaacgc aggacctcca ctcctcttct cctcaacacc cacttttgcc atcgaaaaac     240

cagcccagtt attgggcttg attggagctc gctcattcca attccttcta ttaggctact     300

aacaccatga ctttattagc ctgtctatcc tggcccccct ggcgaggttc atgtttgttt     360

atttccgaat gcaacaagct ccgcattaca cccgaacatc actccagatg agggctttct     420

gagtgtgggg tcaaatagtt tcatgttccc caaatggccc aaaactgaca gtttaaacgc     480

tgtcttggaa cctaatatga caaaagcgtg atctcatcca agatgaacta agtttggttc     540

gttgaaatgc taacggccag ttggtcaaaa agaaacttcc aaaagtcggc ataccgtttg     600

tcttgtttgg tattgattga cgaatgctca aaaataatct cattaatgct tagcgcagtc     660

tctctatcgc ttctgaaccc cggtgcacct gtgccgaaac gcaaatgggg aaacacccgc 720

tttttggatg attatgcatt gtctccacat tgtatgcttc caagattctg gtgggaatac 780

tgctgatagc ctaacgttca tgatcaaaat ttaactgttc taacccctac ttgacagcaa 840

tatataaaca gaaggaagct gccctgtctt aaaccttttt tttatcatca ttattagctt 900

actttcataa ttgcgactgg ttccaattga caagcttttg attttaacga cttttaacga 960

caacttgaga agatcaaaaa acaactaatt attcgaaacg 1000

<210> 42
<211> 32
<212> DNA
<213> Artificial Sequence

<220>
<223> Primer 28

<400> 42
ttaggcgcgc ccatgttggt attgtgaaat ag 32

<210> 43
<211> 35
<212> DNA
<213> Artificial Sequence

<220>
<223> Primer 29

<400> 43
ttattaatta acgttacact caaagtaatg aacac 35

<210> 44
<211> 100
<212> DNA
<213> Artificial Sequence

<220>
<223> Primer 30

<400> 44
acgactttta acgacaactt gagaagatca aaaaacaact aattattcga aacgggtacc 60

cctagggata tcacgcgagt gttatgtcaa gttctggacc 100

<210> 45
<211> 24
<212> DNA
<213> Artificial Sequence

<220>
<223> Primer 31

<400> 45
gaaaacgtac tcagtggttt tctc 24

<210>  46
<211>  1106
<212>  DNA
<213>  Ogataea angusta

<400>  46
acgactttta acgacaactt gagaagatca aaaaacaact aattattcga aacgggtacc      60

cctagggata tcacgcgagt gttatgtcaa gttctggacc tgcgattgag gtgtccacag     120

aattgaagca gaacgttttt ggcgaagacc cagtgatacc cgaatttgcc aaagataccc     180

aatctggcag ttctttcgca aacattactc agtggaaaga gctcggtgca tatttagagg     240

gcttacaaag aatcacacct ggttcctctg actcagaaaa cagcgatcta caatccaaaa     300

atccgctctc cagaccggtt gtgtatcgta tttgcaacca ttgcgacaga cctatcttgg     360

agaaatattt agcagatcat ctcaagagct gcgagcagga aaaacgtaac aaaaccttaa     420

cggcagaaaa atccaatacc gccattacga ataacgccat gaagaaacgg cggttggacg     480

aagctgcaga aaatctgtca agctcactta actctccgga tgttgaggaa aacaaaatga     540

acggtgccaa cggggcgtcc aaaaagcaga aagtcgcgaa agtgcccaag gaaaagaaag     600

tcaggaaaag cgtcaagcca aaaattaccg gaaagccaaa gggacctgtt gatgtggaga     660

aacagtgtgg tgtcccgctg ccgaacggtg gtttctgtgc gcggtccttg acctgtaaga     720

cgcattccat gggagccaaa cgagccgttc cggggagaag cgcgccgtac gaccagctgc     780

ttgctgcgta tcagcgcaag aaccaggcta aaatcggtgc tgctgctgct gctgctcagc     840

aagctcaaga cgaccttatg catggttcgt ctgttccgct ggacgacgaa gaggagacac     900

atcaggtttt ggatggcgtg acgagatcga cgccatggcc tttggagcgg aaagtgataa     960

tgcctactaa aatacgcaac tcatttattc gcatgagaga aatgtttgct ggtgctatac    1020

tgcctagaat gccgtccaat ccgctgggac aaatgcaagg gcgtactgcg gtcgtggaca    1080

cagagaaaac cactgagtac gttttc                                         1106


<210>  47
<211>  23
<212>  DNA
<213>  Artificial Sequence

<220>
<223>  Primer 32

<400>  47
tattcgcatg agagaaatgt ttg                                              23


<210>  48
<211>  25
<212>  DNA
<213>  Artificial Sequence

<220>
<223>  Primer 33

<400>  48
agatctctca cagatactgt ttgtg                                              25


<210>  49
<211>  2503
<212>  DNA
<213>  Ogataea angusta

<400>  49
tattcgcatg agagaaatgt ttgctggtgc tatactgcct agaatgccgt ccaatccgct        60

gggacaaatg caagggcgta ctgcggtcgt ggacacagag aaaaccactg agtacgtttt       120

ccctgtgcgg tctcagcatc aacggatggc accctcgacg caggtgcggt cggccgcacc       180

tatggccaag ccaagtcccc agggtgccgt tgatgttcat tcgcaaatct cggcccaggc       240

ccagctgctc gctcagcagc agcagcagct cagacttgcc caggccaaga aacagcagca       300

acaagcacag atgcaagcta aataccaggc ccagcagctg gctgcagggg tcgttcctca       360

gcaggataaa cacatgcaat tgactccgca gcaacagcag cagatgatga tgaggcggcg       420

gatgtatttg caacagcagt cgcaacaact gcggcaacaa cagcagcagt atattaaccc       480

ccagtacaag agtcaacagg agatgatgat gaatcaacgc cagcaatagt gctgcaacag       540

gctggatcta cgatcttggg gttttcgggt ctgtcgaatc gtcttccagt tgcgaagctc       600

tcggcttggg ttgtaagggc tcaatacgat ctgaaaatct gattctgaaa aaggaggggt       660

gtttccagtt cccgttttca tcgaagttct ccggactagg cggtgtgtat ttgatgagct       720

gatattcttt aagaacatag aagctcatga gaaacccgcc tagaactccg atgaccacaa       780

cgttaaatcg tccgttaact gttttttgta ccatttggta atttgttttt ctaataaaac       840

tccactttta aaaagaagta atctttattt tcttatcatg tcacttccaa agagaattat       900

tagagtaagt aaacaaacat gttcacgtgc taataccagg aaactgaacg gctagtatca       960

gaccccgtgc cgggtatcac agcagtgcca catgaaggca acttgcgcta cttcaacgtg      1020

accatcgacg gtccctccgg ttcgccatac gagggaggca aattcaagct ggagttgttt      1080

ctgccggacg actacccaat gctggcccct aaagtgcgtt ttttgaccaa aatataccat      1140

ccgaacattg acagactggg aagaatctgt ctcgacgtcc taaaggccaa ctggtctcca      1200

gcattgcaag tgcgcaccat tttgctgtcc atccaagcac ttctgagctc gccaaaccca      1260

gacgacccac tagcgaacga ggtggctgag gaatggaaaa ataacgagca aaatgccatc      1320

aaaaccgcca gagagtggac tgaaagattt gcacagtgat tacaacctct aaatacgagc      1380

ttcgcgaatt ccaccggtga gcaacacttc accttcccac acacttgcca gcacgtcact      1440

gaagtcgtca acatacacaa tttcgagtcc caaagtctga tagacccact tttcaggttc      1500

tccagcgtgc gtgcggccct ccttaagaag ctgctcttcc tcgtcgagga atttagcaaa 1560

aagctctttg gctagctccc tgttcctcag attatagatg aagtcttcaa tcacatcctt 1620

gcggttctgc cgtggaagta gcaccttgtt caccttgcct gcaaggtgtg cacccagtag 1680

tttttctctc actcctccga ttgggagaac tttaccagta agcgtgattt ctcctgtcat 1740

ggcgagcgtt tccggcactt ttttgcgcaa aaccagggac atcaaacaca gtgtcatggt 1800

gattcccgcc gaaggcccat ctttcgagat agcaccttcg ggaacgtgca aatgaacgct 1860

agtgttctca tatcgtttga gcagttcgtc tctaaattct tgagtgccgg ccaccaaatt 1920

gttgtgtagc aggtatccaa caagggtatt tgctatctgg gagctctcca acaacacctc 1980

tcccaggcgt ccagtgcagt tcattttctg cgatccagga agaccgatca tctcaaatcg 2040

taaaagagat cccgacccgt cagagttata ggataatccg ttgaccacgc cgtacccttt 2100

ctgaaatttg gtctcacctg ccgctgtagc acgaagatgt gacggactgc caatatattt 2160

tgcgagatcg tgcacagtca ccgttttggt ggcctcgccc atttgcctct caacagcctt 2220

tcctcgacag atagccgcaa tcagccgttc aagatttcga attcctggct cgctggtgta 2280

gtgcgtggcc atcttcagga tcgtctcgtc gtccatggcc accgcgtcac tagaaagtcc 2340

tgcacgttcc aattggcgcg gaataaggta tttcttggaa atctcaactt tctccatata 2400

attgtatcct gcaagttcaa ttacctccat cctgtcacgc aaaggatcac ttagctgcca 2460

tagatcattc gaggtgcaca caaacagtat ctgtgagaga tct 2503

<210> 50
<211> 25
<212> DNA
<213> Artificial Sequence

<220>
<223> Primer 34

<400> 50
ctttctccat ataattgtat cctgc 25

<210> 51
<211> 23
<212> DNA
<213> Artificial Sequence

<220>
<223> Primer 35

<400> 51
ctacacggat tctgtcgaga tac 23

<210> 52
<211> 2567
<212> DNA
<213> Ogataea angusta

<400> 52
ctttctccat ataattgtat cctgcaagtt caattacctc catcctgtca cgcaaaggat 60

cacttagctg ccatagatca ttcgaggtgc acacaaacag tatctgtgag agatctatag 120

ggaagccgat gtagtggtcg tggaagttgg tgttttgttc agggtccaga atttccagca 180

gagcagcctc agggtctcct ttccgagagt tggatccgat cttgtccacc tcgtccaata 240

aaatcaccgg attcattgac tgagagcgcc gcagggcctg gactattagc cctggaattg 300

caccaacata agttcttctg tggcccttca ggtctgcaaa atcatttagt ccaccaacac 360

tgatcctctg gaactttcgc cccagagtgg tggcaattga ccgcgccaat gacgttttcc 420

caacgccggg agggcctgtg agaagcaaga ttggcgcttt cagggtggaa gccgggcgct 480

ctttcgttgt aggctcgtct ccagtgccgt atacaaattc cggctcgtgt ctcggctgtt 540

tctgattgtg cagattcaac actgccagat actctagtat tctttccttg gcactctcca 600

tgccgtaatg atcggcgtcc agttgctctc gcgcacgtgc aaggtccaca ttgactgatt 660

ccacaaaaga gtcgagccgc tgccacggaa gatccatgat aatctccaga tacgctctca 720

atacctggta atccgaggaa ctggactgca tctgtttgag tctctcaaaa tccttcgcaa 780

taagtctctt accgtcctca ttaatcagat atttgtccaa agagtcgaca aaatcctgga 840

tactcttatt ttcgtcattg tcctcagccg ttctgctccc acggccagtg acccgcatga 900

gacccatctc ctcaactagc cgacgcaaat tccgcagatg atttgagata aattgtgagc 960

gcgacacatt gctctgagcc ttgctgctat ggtgatcaag actgctccaa atctccgcca 1020

aatagctagt atcgagatgt tcttcaaaca ccttgttcgc gaacgccacg catttgttaa 1080

atacttcgat acgttctgct cctttggacc tcagatacga aatcttcagc tcaattggaa 1140

atggaaacag cgcaacaaga acgtcgttgt agtgagcaaa gtctcctgac ttcctcccgt 1200

acgcttgtct cagtcgtttc agacctgcag acacgtctga cgccgacagc tgagcattca 1260

aaagcgttgc aagaggcgtg agtcgcaaaa gaatgtgccg cgagcgttcc tccttgtcct 1320

ctgagtccaa agtgccctcg tccttgtagt aggttaggaa ctggtcgatg ttgtcgaata 1380

aacggatcat gtctttcgtg tcgggatgtg gcacagacgt ttcctcctga atctcaacgg 1440

ttgccgtatc ctcgtcggat tgcacaatat agccccgcgt aacgcccttg aaactcacca 1500

cggtcgtctc gctcatctcg taaaagccgt tcaccacaca gacggtgcat gtgtccgagt 1560

ctggatagcc aggcacacag gcaactaaat gtttagaaga aagaccaaag cgcgaagcaa 1620

ttgccgcaac acgttctgag cgttccttag ctgaccatct cgaagccaga tcaagagctt 1680

tttgcttatc gaaaacaaca cgataagtga tgccgggaag aaaaatgagg ttgcgctcca 1740

accggtggac aggaagttcc acttgccacg ctgaagactt ggaagacatg ctagtggaat 1800

atatctgact gaaggctact tttggcgaga taatatatag attatcggat aagctttcct 1860

aatggagcag gagtcgcaca cgacttatat tttttgcttt agctatacaa caacagagaa 1920

atgaatactt atgccaacat gccaacactc tagaacaagc aatgatacca cccgaagcac 1980

tggctcactt gatgaggtcc aaaatttgct gcgtcaggtt ggatatctca tctgccccgt 2040

tgaaatcttc gccggttaga ttgttgaggt tcatcttgcc tctgttgatt tcgcttttta 2100

ttttggactt gatctcctgg ttatcggatt ctagtaactg cagcatcgtc cacaaggcaa 2160

tgtgctcgaa tgtagagttg cctgagttca gaaaatcgga gatgaagcta gagatgccgt 2220

cgtaattttc caaaattatc gtataatctt gtattcttga gcacaagttg gccagggcag 2280

cggccgagtt accgctcact tcagggttcg acgatttggt aagcgggatc aacacgtcca 2340

tgataccaag ctcaagcagc ttagccttga ggtcatctgc cagcgcgaga attgcaaaac 2400

atgcagatat ttcgctttgg acgctttcgg gagcttgcaa caccaactct ttgcatttct 2460

taaccgcgcc cgcttccagt agctccatgc gatttctttc actgctggca gcaaggttcc 2520

tcaaagtgga cacggcgtgg cattgtatct cgacagaatc cgtgtag 2567

<210> 53
<211> 22
<212> DNA
<213> Artificial Sequence

<220>
<223> Primer 36

<400> 53
caacaccaac tctttgcatt tc 22

<210> 54
<211> 23
<212> DNA
<213> Artificial Sequence

<220>
<223> Primer 37

<400> 54
agtaggccag tttatcgtac agg 23

<210> 55
<211> 2488
<212> DNA
<213> Ogataea angusta

<400> 55
caacaccaac tctttgcatt tcttaaccgc gcccgcttcc agtagctcca tgcgatttct 60

ttcactgctg gcagcaaggt tcctcaaagt ggacacggcg tggcattgta tctcgacaga 120

atccgtgtag tccaccaaat taaccagagg cttcaagaaa cctgcgtcga tgatcaatgc 180

ctcgttcata gggtggatcg atatattgcg gatgcatgcg acggcagcaa gaaccaatgg 240

ttcgtgctgc gacttcagca acgagacgag attcggcaag ccacccgcgc gcacgatctc 300
caattggtac aatgcatcgg aggctaggtt tctgagagcc aacgtggcct ggcattggac 360
cctcgggctc gaagagtcca ttagttgcac caactggctg acaagctttg gctcggtggt 420
ggccagtttt tttctattgg actcgtccac agcaatgttg gacaatgctg tcgtacaata 480
gtactggacg tctgggtcag aggaggaaag cagctgaacc agaactggca ccgacccggc 540
ctccacaagc tcttttctgt tttctagaga atgagtcatg ttcagcaggg cacctgtggc 600
attccgttga accctgaggt ctggggactt ggcgagtttc gtgagtggaa taagagcacc 660
agaggtggca attttggtct tgttcttatc ctgggtagcc agatttgtga tacagccaac 720
agcattgcac tggacctcaa tgttggaact catcatctgc ctgataagag gcactagtcc 780
gcccatctcc acaattagga ccttgttgtc atcgttgact gccaggttac ctaacgcggc 840
acaggctgcc cgttggacgt cctggtcaga gctctgtagc aatattaaaa tcggctccaa 900
cacgtctctc gagacggcgc ggatgtcttt ctctgttatt tcggcaaatg ccaaagctgc 960
acttctctgg aggtcgatat tgtccgagta cacgagcgtg ctaagtgctt tcagcggacc 1020
atcagagaaa aagtccacct cgccacggtt ctccaagtat tgcaagagag cggaaatcgc 1080
gtctctctcg ttgtcagcta gaagttgggg atacaagttg tcatgctcac ttttgctaca 1140
acaacagccc atttttatgt gttatttcca attgaagacg ctcttcttat ttgttgggta 1200
tgttttcgca gaagtgtcta tattggcctc aataatttgt tcggttatag gtttgccata 1260
agatgtaatc ttacacgtct tatccgtttg ttctgtttaa acaaattttt atgagggcaa 1320
gatggtagtg agagacacaa gataagatac aaaaaatgtc ttctagattg gtccagcgtg 1380
ctattttcaa gtcgactcgc gagcctacgg gcttgttttt ggagattacc gcatggagct 1440
gtttggtgtt tagcaattat tacccattaa ttttcgttca gtacatactc gcaaagttct 1500
gcctcttcgg ctttactgac atggcaggtt ttcgttcctg cttcacaaac ggatcggcct 1560
tttcgtggta cggcctgttt ccaggcctac tatctttcgc gcgtgtagga gacttttccc 1620
tggcgtcctt ttggttcctg agtctttcgc ggtactcttc cactttcttg tcgatctcgc 1680
tgtcttcaag cccgtcatcc tcaagcttct ctctgtattc cattactttt atctccaact 1740
gtcgtttgcg ctcgtgaagg tcgatatctc tgtctcgaac agtagtggca acctgtttgc 1800
gctcgttttt ctgccgaagt tgccgcctca aatactgctt ccccccactc tcatctatac 1860
gcgcattgcc gtgcaagttg ctgaggttac gctgcacgta tccgtttgtt ccagagcccc 1920
tcgcggtgct cagaccgatt ccattgtaca tttaaatttt atttaatttt tgttggcggt 1980
gcaaatcgtt ttagcatgtc gcaacagatg ctgagtcgct ggattaagcg ggagaacagt 2040
tcatctcccg tgccagacac ggccaacaac acagaggcgg tggaaaacag ccagaagaac 2100

ggcaagtcgc gttccacgcc gtcgactggg tccaaagcgt cgtccataga cgcgttcagg 2160

tattccgaca ctgctggatc gctgacccgc tccaaatttg cagataagct taagggatac 2220

actgttggga cccggaaaag gctgtttagc gaagaaggcg aggatgcccc gaaaaaggcg 2280

aaatcaagca agttgacccc actcgaacag cagatctacg atctcaaaat gcaacataga 2340

gacaaactgc tggccattca agttggctac aaatacaagt tctatggcga ggatgctcgt 2400

gctgcgtcgc aaattttgaa catcatgttc attccaggac ggctgttttt caccaacagc 2460

gatgacctgt acgataaact ggcctact 2488

<210> 56
<211> 25
<212> DNA
<213> Artificial Sequence

<220>
<223> Primer 38

<400> 56
tcaagttggc tacaaataca agttc 25

<210> 57
<211> 70
<212> DNA
<213> Artificial Sequence

<220>
<223> Primer 39

<400> 57
agatggctac ctgattggac gaggacacca agacatttct acaaaaaaaa tgtgatcaca 60

ccagatcttg 70

<210> 58
<211> 2624
<212> DNA
<213> Ogataea angusta

<400> 58
tcaagttggc tacaaataca agttctatgg cgaggatgct cgtgctgcgt cgcaaatttt 60

gaacatcatg ttcattccag gacggctgtt tttcaccaac agcgatgacc tgtacgataa 120

actggcctac tgctctattc ccgacgtgag acttcatgtt cacctgaaac ggttgctgag 180

tgccggctac aaagttgccg ttgttgacca gaacgagaca gcagccatca agtccaccac 240

agcgtcgaaa aacaagctgt ttgagcgacg catcagtaaa gtgtacactt cgtccacgta 300

catcgacaac gaagacgtga tttctggggg ccggttcgtt gtcgcgttga cagagactaa 360

gaacaaggaa acccctgtga tatctctggt tgccgttgac gtttactctg cagacatcat 420

atatgacgag tttgaggaca attttgtgcg caacgagctg gaaactaggc tttaccatct 480

cgatccaacg gaatttctat taatcggaga gatttcgcgt gaaacacaga aagcgttgga 540
tctgtttaaa cggtacactc gtgagtcggc aactagcttg cggtcggagg ccagggatgc 600
aaagacatac acgcagatcg ccgatgttct gaacgtcaac ctatccgacg aggcctttga 660
tctcgtcgca aagctttcag tcgctgtcca aggttgtttt gcagagctcg tcgagcacat 720
gatcgagttc gaattggcca acgtgtttga tctggtggac aaatacaccc atttttccag 780
cgtccatcga tgcatgattt tggacgcaaa cactctgcgt aacctcgaaa tatacaaaaa 840
cagcaccaat ggccaggagt acggctctct gctgtggatg ctggaccata caaataccca 900
gttcggacgc agagagctaa gacgatggat cggcaggcca ttgacggaca gagaagaggt 960
ggccaagaga gcagactccg tggagagcat tatgaaaaac taccagtccg tggccatcga 1020
gtcgaccgtc aagcttttgc gcaattgtcc ggacctggag gcggcgctca gtcgtatcca 1080
ctacggccgg tccaaacgca aggacactta tatgtttctc aaaaaaatga acgagattct 1140
ccagttctac ggagacctcc cagacaccta tgctacctct gtgcagacca acccgtctct 1200
acgggagata ttcgatgatc tcaaaacttc agcctcctct gggctcaaag acttcaggaa 1260
tttgctggac atggtccact cgccagcagc catcgacgac acgagccccg agcacgtgac 1320
cggctatttc aacaccagct tctttgatta ccacctgatc caacagcatc tggagaacat 1380
ttctcaggtg gagcagcagc tggaagccga actcaaggaa atccgtaaaa tcgttggccg 1440
tccaggcatg ggctatgtga ccaacaacaa ggagccgtat ttggtggagg tgcgcaacac 1500
tcaggttgcc agcctgccga aagactggct taaaatcaac ggcaccaagt ctgtgtcgcg 1560
gttcagaact ccttcgggag ccgcactcta cagacagatc cagtaccact cagagatgct 1620
ccagaaagag tgcaacgact gcttcaccaa gtttgtcaaa agaatcgacg aatactatct 1680
cgacttgaac aaaactattc gacatctcgc tgttctagac tcactaattt ccctgagtgc 1740
cgcctcctcg ctcaacgaag gctatacaaa acctgtgttt gtggactctc cgtgcattga 1800
tgtgaaaaac tctcgcaacc caatttcgga aaacctcaag acatcaactc gttacattcc 1860
caacgatttc aagatgtcgc actcagaagg cagaatagcc ttgatcacag gtccgaatat 1920
gggaggaaag tcgtctttca tccgccagat cgcactgctt gtggtcatgg cacagatagg 1980
ctgctacata cctgcggact ccgggtccaa gctgagcatt ttcgactcga tccacacccg 2040
aatgggcgca caagacgata ttatcaaggg agagtctacg tttcaggtgg agctgaagga 2100
atgcagcacc attttgaaag agtgcggacc acggtcgctg gttctgatgg acgaagtggg 2160
ccgcggaacc agtaccatgg acggcttcgc catagcgcat tccattctgc ggtaccttgt 2220
tacagatagg tcgccgtttg tactattcat tacacactat cagaacctga ggtcgtttga 2280
gaggttcaaa gaggtgaaaa gttaccatat gggcatccaa aaagtggatg aagacatcgt 2340
cttcacatat aagctatccg caggatgctc tgatcgttca tacggtatca attgtgcaaa 2400

gttggcagga ctgcctaaac ctgttcttga atccgcacat cagaactcag tcaggttcga 2460

aaatgactgg agactcaagg aggcactgag tctggcacac aactttcgca ctctcataga 2520

aaataaagat tatgccaagc tgctggaact ggcacaagat ctggtgtgat cacatttttt 2580

ttgtagaaat gtcttggtgt cctcgtccaa tcaggtagcc atct 2624

<210> 59
<211> 70
<212> DNA
<213> Artificial Sequence

<220>
<223> Primer 40

<400> 59
ttatgccaag ctgctggaac tggcacaaga tctggtgtga tcacattttt tttgtagaaa 60

tgtcttggtg 70

<210> 60
<211> 70
<212> DNA
<213> Artificial Sequence

<220>
<223> Primer 41

<400> 60
caaagtagta accaaagttg gccatggaac tggcaactta ccagtagtac agatgaactt 60

caaagtcaac 70

<210> 61
<211> 735
<212> DNA
<213> Artificial Sequence

<220>
<223> PgapGFP1

<400> 61
ttatgccaag ctgctggaac tggcacaaga tctggtgtga tcacattttt tttgtagaaa 60

tgtcttggtg tcctcgtcca atcaggtagc catctctgaa atatctggct ccgttgcaac 120

tccgaacgac ctgctggcaa cgtaaaattc tccggggtaa aacttaaatg tggagtaatg 180

gaaccagaaa cgtctcttcc cttctctctc cttccaccgc ccgttaccgt ccctaggaaa 240

ttttactctg ctggagagct tcttctacgg cccccttgca gcaatgctct tcccagcatt 300

acgttgcggg taaaacggag gtcgtgtacc cgacctagca gcccagggat ggaaaagtcc 360

cggccgtcgc tggcaataat agcgggcgga cgcatgtcat gagattattg gaaaccacca 420

gaatcgaata taaaaggcga acacctttcc caattttggt ttctcctgac ccaaagactt 480

taaatttaat ttatttgtcc ctatttcaat caattgaaca actatcaaaa cacaactagt 540

atggtttcta agggtgaaga gttgttcact ggtgttgttc caatcttggt tgagttggac 600

ggtgacgtta acggacacaa gttctctgtt tctggtgaag gtgagggtga cgctacttac 660

ggaaagttga ctttgaagtt catctgtact actggtaagt tgccagttcc atggccaact 720

ttggttacta ctttg 735

<210> 62
<211> 70
<212> DNA
<213> Artificial Sequence

<220>
<223> Primer 42

<400> 62
gtgagggtga cgctacttac ggaaagttga ctttgaagtt catctgtact actggtaagt 60

tgccagttcc 70

<210> 63
<211> 100
<212> DNA
<213> Artificial Sequence

<220>
<223> Primer 43

<400> 63
caattcaatc agataattaa taatatcaaa gttctatcaa aatacatgta ttataccccc 60

gatgatgtac aacgcgttac ttgtacaact cgtccatacc 100

<210> 64
<211> 696
<212> DNA
<213> Artificial Sequence

<220>
<223> GFP2

<400> 64
gtgagggtga cgctacttac ggaaagttga ctttgaagtt catctgtact actggtaagt 60

tgccagttcc atggccaact ttggttacta ctttgactta cggtgttcag tgtttctcca 120

gatacccaga ccacatgaag cagcacgatt tcttcaagtc tgctatgcca gagggttacg 180

ttcaagagag aactatcttc ttcaaggacg acggtaacta caagactaga gctgaggtta 240

agttcgaggg tgacacattg gttaacagaa tcgagttgaa gggtatcgac ttcaaagagg 300

acggaaacat cttgggtcac aagttggagt acaactacaa ctcccacaac gtttacatca 360

tggctgacaa gcagaagaac ggtatcaagg ttaacttcaa gatcagacac aacatcgagg 420

acggttccgt tcaattggct gaccactacc aacagaacac tccaattggt gacggtccag 480

ttttgttgcc agacaaccac tacttgtcca ctcaatccgc tttgtccaag gacccaaacg 540

agaagagaga tcacatggtt ttgttggagt tcgttactgc tgctggtatc actttgggta 600

tggacgagtt gtacaagtaa cgcgttgtac atcatcgggg gtataataca tgtattttga 660

tagaactttg atattattaa ttatctgatt gaattg 696

<210> 65
<211> 24
<212> DNA
<213> Artificial Sequence

<220>
<223> Primer 44

<400> 65
gcttactttc ataattgcga ctgg 24

<210> 66
<211> 76
<212> DNA
<213> Artificial Sequence

<220>
<223> Primer 45

<400> 66
   tgagcggata acaatttcac acaggaaaca gctatgacca tgattacgcc ttttttgtag 60
   aaatgtcttg gtgtcc 76

<210> 67
<211> 70
<212> DNA
<213> Artificial Sequence

<220>
<223> Primer 46

<400> 67
ttctcgtaag tgcccaactt gaactgagga acagtcatgt ctaaggttac ttgtacaact 60

cgtccatacc 70

<210> 68
<211> 1309
<212> DNA
<213> Artificial Sequence

<220>
<223> Fragment G1

<400> 68
   tgagcggata acaatttcac acaggaaaca gctatgacca tgattacgcc ttttttgtag 60
   aaatgtcttg gtgtcctcgt ccaatcaggt agccatctct gaaatatctg gctccgttgc 120
   aactccgaac gacctgctgg caacgtaaaa ttctccgggg taaaacttaa atgtggagta 180
   atggaaccag aaacgtctct tcccttctct ctccttccac cgcccgttac cgtccctagg 240
   aaattttact ctgctggaga gcttcttcta cggccccctt gcagcaatgc tcttcccagc 300
   attacgttgc gggtaaaacg gaggtcgtgt acccgaccta gcagcccagg gatggaaaag 360
   tcccggccgt cgctggcaat aatagcgggc ggacgcatgt catgagatta ttggaaacca 420
   ccagaatcga atataaaagg cgaacacctt tcccaatttt ggtttctcct gacccaaaga 480
   ctttaaattt aatttatttg tccctatttc aatcaattga acaactatca aaacacaact 540
   agtatggttt ctaaggtgaa agagttgttc actggtgttg ttccaatctt ggttgagttg 600
   gacggtgacg ttaacggaca caagttctct gtttctggtg aaggtgaggg tgacgctact 660
   tacggaaagt tgactttgaa gttcatctgt actactggta agttgccagt tccatggcca 720
   actttggtta ctactttgac ttacggtgtt cagtgtttct ccagataccc agaccacatg 780
   aagcagcacg atttcttcaa gtctgctatg ccagagggtt acgttcaaga gagaactatc 840
   ttcttcaagg acgacggtaa ctacaagact agagctgagg ttaagttcga gggtgacaca 900
   ttggttaaca gaatcgagtt gaagggtatc gacttcaaag aggacggaaa catcttgggt 960
   cacaagttgg agtacaacta caactcccac aacgtttaca tcatggctga caagcagaag 1020
   aacggtatca aggttaactt caagatcaga cacaacatcg aggacggttc cgttcaattg 1080
   gctgaccact accaacagaa cactccaatt ggtgacggtc cagttttgtt gccagacaac 1140
   cactacttgt ccactcaatc cgctttgtcc aaggacccaa acgagaagag agatcacatg 1200
   gttttgttgg agttcgttac tgctgctggt atcactttgg gtatggacga gttgtacaag 1260
   taaccttaga catgactgtt cctcagttca agttgggcac ttacgagaa 1309

<210> 69
<211> 29
<212> DNA
<213> Artificial Sequence

<220>
<223> Primer 47

<400> 69
ccttagacat gactgttcct cagttcaag 29

<210> 70
<211> 21
<212> DNA
<213> Artificial Sequence

<220>
<223> Primer 48

<400> 70
atcgcgattc ttgggtgagg a 21

<210>  71
<211>  584
<212>  DNA
<213>  Artificial Sequence

<220>
<223>  Fragment G2

<400>  71
ccttagacat gactgttcct cagttcaagt tgggcactta cgagaagacc ggtcttgcta      60

gattctaatc aagaggatgt cagaatgcca tttgcctgag agatgcaggc ttcatttttg     120

atactttttt atttgtaacc tatatagtat aggatttttt ttgtcatttt gtttcttctc     180

gtacgagctt gctcctgatc agcctatctc gcagctgatg aatatcttgt ggtaggggtt     240

tgggaaaatc attcgagttt gatgtttttc ttggtatttc ccactcctct tcagagtaca     300

gaagattaag tgagaagttc gtttgtgcaa gcttagatct tctagagaca ataagaagaa     360

aaaaaaagaa aagcggtggg ggagggatta ttaaataagg attatgtaac cccagggtac     420

cgttctatac atatttaagg attatttagg acaatcgatg aaatcggcat caaactggat     480

gggagtatag tgtccggata atcggataaa tcatcttgcg aggagccgct tggttggttg     540

gtgagaggag tgaaatatgt gtctcctcac ccaagaatcg cgat                      584


<210>  72
<211>  20
<212>  DNA
<213>  Artificial Sequence

<220>
<223>  Primer 49

<400>  72
atcagcaccc tgtgggggac                                                  20


<210>  73
<211>  22
<212>  DNA
<213>  Artificial Sequence

<220>
<223>  Primer 50

<400>  73
ggcgtaatca tggtcatagc tg                                               22


<210>  74
<211>  4095
<212>  DNA
<213>  Artificial Sequence

<220>
<223>  Fragment G3

<400> 74
atcagcaccc tgtgggggac actattggcc tccctcccaa accttcgatg tggtagtgct 60
ttattatatt gattacattg attacatagc taaaccctgc ctggttgcaa gttgagctcc 120
gaattccaat attagtaaaa tgcctgcaag ataacctcgg tatggcgtcc gaccccgctt 180
aattatttta actcctttcc aacgaggact tcgtaatttt tgattaggga gttgagaaac 240
ggggggtctt gatacctcct cgatttcaga tcccaccccc tctcagtccc aagtgggacc 300
cccctcggcc gtgaaatgcg cgcactttag tttttttcgc atgtaaacgc cggtgtccgt 360
catctagaga attccccgct cagaagaact cgtcaagaag gcgatagaag gcgatgcgct 420
gcgaatcggg agcggcgata ccgtaaagca cgaggaagcg gtcagcccat tcgccgccaa 480
gctcttcagc aatatcacgg gtagccaacg ctatgtcctg atagcggtcc gccacaccca 540
gccggccaca gtcgatgaat ccagaaaagc ggccattttc caccatgata ttcggcaagc 600
aggcatcgcc atgggtcacg acgagatcct cgccgtcggg catgcgcgcc ttgagcctgg 660
cgaacagttc ggctggcgcg agcccctgat gctcttcgtc cagatcatcc tgatcgacaa 720
gaccggcttc catccgagta cgtgctcgct cgatgcgatg tttcgcttgg tggtcgaatg 780
ggcaggtagc cggatcaagc gtatgcagcc gccgcattgc atcagccatg atggatactt 840
tctcggcagg agcaaggtga gatgacagga gatcctgccc cggcacttcg cccaatagca 900
gccagtccct tcccgcttca gtgacaacgt cgagcacagc tgcgcaagga acgcccgtcg 960
tggccagcca cgatagccgc gctgcctcgt cctgcagttc attcagggca ccggacaggt 1020
cggtcttgac aaaaagaacc gggcgcccct gcgctgacag ccggaacacg gcggcatcag 1080
agcagccgat tgtctgttgt gcccagtcat agccgaatag cctctccacc caagcggccg 1140
gagaacctgc gtgcaatcca tcttgttcaa tcatatggtt tctatattat ctttgtacta 1200
aagagcaatt gataatgtgc gagaaaaact ggtccttata tgccgtttgc agcactccct 1260
cccgaacttt acgaaaagtc gtgcgccacc tgattttcat cacgccaaaa acctacacgt 1320
atgactactc cgggccagtg ttcaccacga gctatatagt gttaattaat taccttattg 1380
gttagctctg catgtaaggg tggtgtgagc cgggaattgg gtctactcta gcgttcagta 1440
aggtgatata aagctctgta gaattcactg gccgtcgttt tacaacgtcg tgactgggaa 1500
aaccctggcg ttacccaact taatcgcctt gcagcacatc cccctttcgc cagctggcgt 1560
aatagcgaag aggcccgcac cgatcgccct tcccaacagt tgcgcagcct gaatggcgaa 1620
tggcgcctga tgcggtattt tctccttacg catctgtgcg gtatttcaca ccgcatatgg 1680
tgcactctca gtacaatctg ctctgatgcc gcatagttaa gccagccccg acacccgcca 1740
acacccgctg acgcgccctg acgggcttgt ctgctcccgg catccgctta cagacaagct 1800
gtgaccgtct ccgggagctg catgtgtcag aggttttcac cgtcatcacc gaaacgcgcg 1860

agacgaaagg gcctcgtgat acgcctattt ttataggtta atgtcatgat aataatggtt 1920

tcttagacgt caggtggcac ttttcgggga aatgtgcgcg gaacccctat ttgtttattt 1980

ttctaaatac attcaaatat gtatccgctc atgagacaat aaccctgata aatgcttcaa 2040

taatattgaa aaaggaagag tatgagtatt caacatttcc gtgtcgccct tattcccttt 2100

tttgcggcat tttgccttcc tgtttttgct cacccagaaa cgctggtgaa agtaaaagat 2160

gctgaagatc agttgggtgc acgagtgggt tacatcgaac tggatctcaa cagcggtaag 2220

atccttgaga gttttcgccc cgaagaacgt tttccaatga tgagcacttt taaagttctg 2280

ctatgtggcg cggtattatc ccgtattgac gccgggcaag agcaactcgg tcgccgcata 2340

cactattctc agaatgactt ggttgagtac tcaccagtca cagaaaagca tcttacggat 2400

ggcatgacag taagagaatt atgcagtgct gccataacca tgagtgataa cactgcggcc 2460

aacttacttc tgacaacgat cggaggaccg aaggagctaa ccgctttttt gcacaacatg 2520

ggggatcatg taactcgcct tgatcgttgg gaaccggagc tgaatgaagc cataccaaac 2580

gacgagcgtg acaccacgat gcctgtagca atggcaacaa cgttgcgcaa actattaact 2640

ggcgaactac ttactctagc ttcccggcaa caattaatag actggatgga ggcggataaa 2700

gttgcaggac cacttctgcg ctcggccctt ccggctggct ggtttattgc tgataaatct 2760

ggagccggtg agcgtgggtc tcgcggtatc attgcagcac tggggccaga tggtaagccc 2820

tcccgtatcg tagttatcta cacgacgggg agtcaggcaa ctatggatga acgaaataga 2880

cagatcgctg agataggtgc ctcactgatt aagcattggt aactgtcaga ccaagtttac 2940

tcatatatac tttagattga tttaaaactt catttttaat ttaaaaggat ctaggtgaag 3000

atcctttttg ataatctcat gaccaaaatc ccttaacgtg agttttcgtt ccactgagcg 3060

tcagaccccg tagaaaagat caaaggatct tcttgagatc ctttttttct gcgcgtaatc 3120

tgctgcttgc aaacaaaaaa accaccgcta ccagcggtgg tttgtttgcc ggatcaagag 3180

ctaccaactc tttttccgaa ggtaactggc ttcagcagag cgcagatacc aaatactgtc 3240

cttctagtgt agccgtagtt aggccaccac ttcaagaact ctgtagcacc gcctacatac 3300

ctcgctctgc taatcctgtt accagtggct gctgccagtg gcgataagtc gtgtcttacc 3360

gggttggact caagacgata gttaccggat aaggcgcagc ggtcgggctg aacggggggt 3420

tcgtgcacac agcccagctt ggagcgaacg acctacaccg aactgagata cctacagcgt 3480

gagcattgag aaagcgccac gcttcccgaa gggagaaagg cggacaggta tccggtaagc 3540

ggcagggtcg gaacaggaga gcgcacgagg gagcttccag ggggaaacgc ctggtatctt 3600

tatagtcctg tcgggtttcg ccacctctga cttgagcgtc gatttttgtg atgctcgtca 3660

ggggggcgga gcctatggaa aaacgccagc aacgcggcct ttttacggtt cctggccttt 3720

tgctggcctt ttgctcacat gttctttcct gcgttatccc ctgattctgt ggataaccgt 3780

attaccgcct ttgagtgagc tgataccgct cgccgcagcc gaacgaccga gcgcagcgag 3840

tcagtgagcg aggaagcgga agagcgccca atacgcaaac cgcctctccc cgcgcgttgg 3900

ccgattcatt aatgcagctg gcacgacagg tttcccgact ggaaagcggg cagtgagcgc 3960

aacgcaatta atgtgagtta gctcactcat taggcacccc aggctttaca ctttatgctt 4020

ccggctcgta tgttgtgtgg aattgtgagc ggataacaat ttcacacagg aaacagctat 4080

gaccatgatt acgcc 4095

<210> 75
<211> 20
<212> DNA
<213> Artificial Sequence

<220>
<223> Primer 51

<400> 75
ttcgtcaacc tcttgcggag 20

<210> 76
<211> 29
<212> DNA
<213> Artificial Sequence

<220>
<223> Primer 52

<400> 76
gctttatata tgggagaaag ttaactacg 29